(19) Europäisches Patentamt
European Patent Office
Office européen des brevets

(11) Veröffentlichungsnummer: **0 152 596**
**A1**

(12) **EUROPÄISCHE PATENTANMELDUNG**

(21) Anmeldenummer: **84115443.8**

(22) Anmeldetag: **14.12.84**

(51) Int. Cl.[4]: **C 07 D 405/06**, C 07 D 405/14, C 07 D 413/14, A 61 K 31/41

(30) Priorität: **10.02.84 DE 3404819**

(43) Veröffentlichungstag der Anmeldung: **28.08.85 Patentblatt 85/35**

(84) Benannte Vertragsstaaten: **AT BE CH DE FR GB IT LI LU NL SE**

(71) Anmelder: **LUDWIG HEUMANN & CO GMBH, Heideloffstrasse 18-28 Postfach 2260, D-8500 Nürnberg (DE)**

(72) Erfinder: **Schickaneder, Helmut, Dr. Dipl.-Chem., Moosäcker 25, D-8501 Eckental (DE)**
Erfinder: **Höxer, Klaus, Dr. Dipl.-Biol., Schulstrasse 1, D-8503 Altdorf (DE)**
Erfinder: **Ahrens, Kurt Henning, Dr., Praterstrasse 9, D-8500 Nürnberg (DE)**

(74) Vertreter: **Kraus, Walter, Dr. et al, Patentanwälte Kraus Weisert & Partner Thomas-Wimmer-Ring 15, D-8000 München 22 (DE)**

(54) **Neue 1,3-Dioxolanylderivate, Verfahren zu ihrer Herstellung, ihre Verwendung und diese Verbindungen enthaltendes Arzneimittel.**

(57) Es werden 1,3-Dioxolanylderivate der allgemeinen Formel I

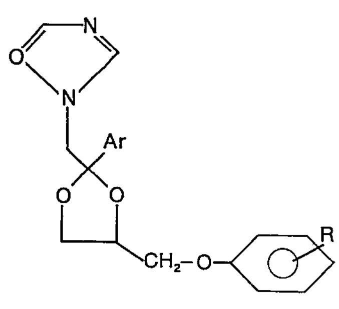

beschrieben. Diese Verbindungen haben eine antimikrobielle Aktivität und können daher mit Vorteil in der Chemotherapie eingesetzt werden.

EP 0 152 596 A1

# BESCHREIBUNG

Gegenstand der Erfindung sind neue 1,3-Dioxolanylderivate der allgemeinen Formel I

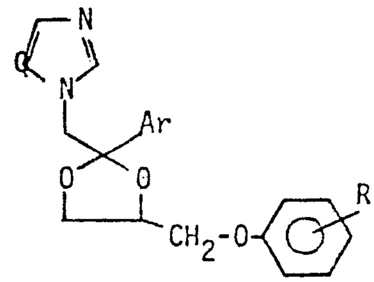

(I)

in der Q für CH oder N steht, Ar eine unsubstituierte oder mit 1 bis 3 Halogenatomen substituierte Phenylgruppe bedeutet. Als Halogensubstituenten der Phenylgruppe kommen Fluor-, Chlor- und Bromatome in Betracht, wobei Chloratome bevorzugt werden. Hierbei ist die Disubstitution bevorzugt, wobei die Disubstitution in 2- und 4-Position besonders bevorzugt ist.

Die Gruppe R hat folgende Bedeutungen:

a) ein Rest der allgemeinen Formel II

N-CN
"
-NH-C-$R^1$ (II)

in der $R^1$ eine unsubstituierte oder substituierte, vorzugsweise mono- oder disubstituierte, Aminogruppe bedeutet.

Bei einer Monosubstitution der Aminogruppe kommen zum Beispiel folgende Gruppen in Betracht: lineare, verzweigtkettige oder cyclische Niedrigalkylgruppen. Hierin werden unter Niedrigalkylgruppen Gruppen mit 1 bis 6, vorzugsweise 1 bis 3, Kohlenstoffatomen im Alkylteil verstanden. Beispiele sind Methylgruppen, Ethylgruppen, Propylgruppen, Isopropylgruppen, Cyclopentylgruppen und Cyclohexylgruppen, wobei Methyl-, Ethyl-, Propyl- und Cyclohexylgruppen bevorzugt werden.

Bei einer Disubstitution der Amingruppe kommen insbesondere lineare Niedrigalkylgruppen mit 1 bis 6, vorzugsweise 1 bis 3, Kohlenstoffatomen im Alkylteil in Betracht, ganz besonders bevorzugt Dimethylgruppen und Diethylgruppen.

Weiterhin kann beispielsweise $R^1$ einen gegebenenfalls substituierten Pyrrolidin-, Piperidin-, Morpholin- oder Piperazinring bedeuten. Ein solcher Ring ist vorzugsweise in 4-Position durch eine Methylgruppe oder Acetylgruppe substituiert. Bevorzugt werden der unsubstituierte Pyrrolidin- und Morpholinring.

Als weitere Monosubstituenten der Aminogruppe kommen unsubstituierte oder substituierte Phenyl- oder Benzylgruppen in Betracht. Vorzugsweise sind diese mit 1 bis 3 Halogenatomen, vorzugsweise Chloratomen, insbesondere mit 1 Halogenatom in 4-Position des Aromaten, substituiert.

Weiterhin kann $R^1$ eine Phenoxygruppe, eine lineare, verzweigtkettige oder cyclische Alkoxygruppe, vorzugsweise Niedrigalkoxygruppe, oder eine Niedrigthioalkylgruppe bedeuten. Hierin werden unter Niedrigalkoxygruppen Gruppen mit 1 bis 6, vorzugsweise 1 bis 3, Kohlenstoffatomen im Alkylteil verstanden. Die $C_1$- bis $C_6$-Kohlenstoffkette kann durch ein eingebautes Sauerstoffatom unterbrochen sein, so daß eine Alkoxyalkoxygruppe gebildet wird. Beispiele für solche Alkoxygruppen sind Methoxygruppen, Ethoxygruppen, Propoxygruppen, Butoxygruppen, Isopropoxygruppen, Cyclohexyloxygruppen oder Methoxyethylgruppen. Bevorzugt werden Phenoxygruppen, Methoxygruppen, Ethoxygruppen, Propoxygruppen, Isopropoxygruppen und Butoxygruppen. Für Niedrigthioalkylgruppen kommen zum Beispiel Thiomethyl- und Thioethylgruppen, vorzugsweise Thiomethylgruppen, in Betracht.

b) ein Rest der allgemeinen Formel III

$$-NH-R^2 \qquad (III)$$

in der $R^2$ einen ein- oder zweifachsubstituierten 5- oder 6-Ringheterocyclus aus der Gruppe 1,2,4-Triazole, 1,2,4-Oxadiazole und Triazine bedeutet, wobei der Heterocyclus mit dem Stickstoffatom des Restes III über ein Kohlenstoffatom verbunden ist.

Als Substituenten kommen die Aminogruppe und Niedrigalkylgruppe, vorzugsweise $C_{1-3}$-Alkylgruppe, in Betracht.

So kann $R^2$ beispielsweise ein 1,2,4-Triazolring sein, der in Position 1 durch ein Wasserstoffatom oder durch Niedrigalkylgruppen, vorzugsweise Methyl- oder Ethylgruppen, und in Position 3 oder 5 durch eine Aminogruppe substituiert ist.

Weiterhin kann beispielsweise $R^2$ ein 1,2,4-Oxadiazolring sein, der in Position 3 oder 5 durch eine Aminogruppe monosubstituiert ist.

Als 6-Ringheterocyclus kommt vorzugsweise ein Triazinring in Betracht, der in den Positionen 4 und 6 durch Aminogruppen disubstituiert ist.

c) ein Rest der allgemeinen Formel IV

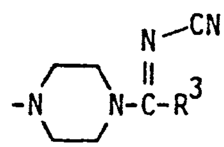

(IV)

in der $R^3$ eine unsubstituierte oder substituierte Aminogruppe oder einen gegebenenfalls substituierten Pyrrolidin-, Piperidin-, Morpholin- oder Piperazinring bedeuten. Ein solcher Ring ist vorzugsweise in 4-Position durch eine Methylgruppe oder Acetylgruppe substituiert. Bevorzugt werden der unsubstituierte Pyrrolidin- und der Morpholinring.

d) ein Rest der allgemeinen Formel V

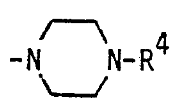

(V)

in der $R^4$ ein ein- oder zweifachsubstituierter 5- oder 6-Ringheterocyclus aus der Gruppe 1,2,4-Triazole, 1,2,4-Oxadiazole und Triazine bedeutet, wobei der Heterocyclus mit dem Stickstoffatom des Piperazinrings über ein Kohlenstoffatom verbunden ist.

Die Erfindung umfaßt auch die physiologisch annehmbaren Salze dieser neuen 1,3-Dioxolanylderivate.

Diese Salze können zum Beispiel mit Mineralsäure, wie Chlor-, Brom- und Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure etc. gebildet werden.

Die Erfindung umschließt weiterhin alle tautomeren und isomeren Formen und deren Salze. Die erfindungsgemäßen Verbindungen können Di- und Trisalze sowie Hydrate bilden, die gleichfalls unter den Rahmen der vorliegenden Erfindung fallen.

Die erfindungsgemäßen Verbindungen können wie folgt hergestellt werden:

$a_1$) Zur Herstellung von Verbindungen der allgemeinen Formel I, bei der R für eine Gruppe der allgemeinen Formel II

$$-NH-\overset{N-CN}{\overset{\|}{C}}-R^1 \qquad (II)$$

und $R^1$ für eine Thiomethyl- oder Phenoxygruppe steht, wird eine Verbindung der allgemeinen Formel VI

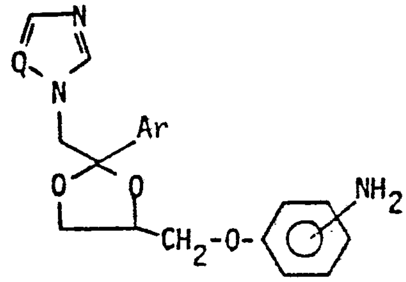

(VI)

mit einer Verbindung der allgemeinen Formel VII

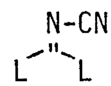

(VII)

in der L eine Thiomethyl- oder Phenoxygruppe als Austrittsgruppe bedeutet, in einem Lösungsmittel zu einer erfindungsgemäßen Verbindung der allgemeinen Formel Ia

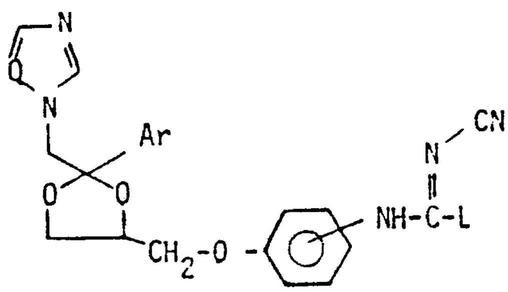

(Ia)

in der L eine Thiomethyl- oder Phenoxygruppe bedeutet, umgesetzt. Die Umsetzung erfolgt in einem inerten Lösungsmittel, wie Methanol, Ethanol oder Isopropanol, bevorzugt Isopropanol, bei Raumtemperatur bis Rückflußtemperatur des Lösungsmittels und unter Anwendung von beispielsweise äquimolaren Mengen. Die als Ausgangsverbindung eingesetzte Verbindung VI kann in an sich bekannter Weise hergestellt werden (J. Med. Chem. 22, 1003 (1979)).

Die Aufarbeitung und Isolierung erfolgt in üblicher Weise. Die erhaltene Verbindung kann gegebenenfalls in ihr physiologisch annehmbares Salz umgewandelt werden.

$a_2$) Zur Herstellung von Verbindungen der allgemeinen Formel I, in der R für eine Gruppe der allgemeinen Formel II

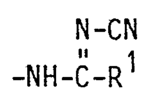

(II)

und $R^1$ für eine unsubstituierte oder substituierte Aminogruppe steht, wird eine Verbindung der allgemeinen Formel Ia mit einer

entsprechend substituierten Aminoverbindung in einem Lösungsmittel umgesetzt. Als Lösungsmittel wird beispielsweise ein Alkohol, wie Methanol, Ethanol oder Isopropanol, bevorzugt Isopropanol, verwendet. Es wird vorzugsweise bei Temperaturen von Raumtemperatur bis Rückflußtemperatur des verwendeten Lösungsmittels und unter Anwendung von beispielsweise äquimolaren Mengen gearbeitet.

Die Aufarbeitung und Isolierung erfolgt in üblicher Weise. Die erhaltene Verbindung kann gegebenenfalls in ihr physiologisch annehmbares Salz umgewandelt werden.

$a_3$) Zur Herstellung von Verbindungen der allgemeinen Formel I, bei denen R für eine Gruppe der allgemeinen Formel II

$$-NH-\overset{\overset{N-CN}{\|}}{C}-R^1 \qquad (II)$$

und $R^1$ für eine Alkoxygruppe steht, wird eine Verbindung der allgemeinen Formel Ia mit einem Alkalialkoholat in dem entsprechenden Alkohol als Lösungsmittel umgesetzt. Die Umsetzung erfolgt bei Raumtemperatur bis Rückflußtemperatur und beispielsweise unter Anwendung äquimolarer Mengen. Als Alkalialkoholat kommt vorzugsweise Natriumalkoholat in Betracht.

Die Aufarbeitung und Isolierung erfolgt in üblicher Weise. Die erhaltene Verbindung kann gegebenenfalls in ihr physiologisch annehmbares Salz umgewandelt werden.

$a_4$) Zur Herstellung von Verbindungen der allgemeinen Formel I, bei denen R für eine Gruppe der allgemeinen Formel II steht,

$$-NH-\overset{\overset{N-CN}{\|}}{C}-R^1 \qquad (II)$$

und $R^1$ die oben angegebene Bedeutung besitzt, wird eine Verbindung der allgemeinen Formel VIII

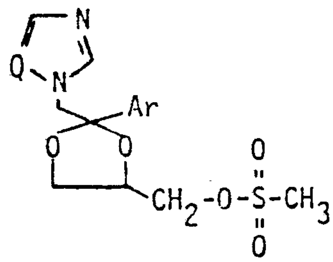

(VIII)

mit einer Verbindung der allgemeinen Formel IX

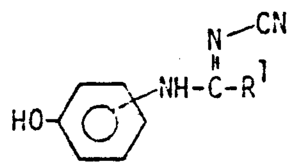

(IX)

in der $R^1$ die oben angegebene Bedeutung besitzt, umgesetzt. Die als Ausgangsprodukt verwendete Verbindung der allgemeinen Formel VIII kann in an sich bekannter Weise hergestellt werden (J. Med. Chem. 22, 1003 (1979)). Vorzugsweise werden äquimolare Mengen der einzelnen Reaktionsteilnehmer eingesetzt. Die Umsetzung erfolgt basenkatalysiert in einem polaren aprotischen Lösungsmittel. Als Basen können zum Beispiel Natriumhydrid oder Kaliumcarbonat, vorzugsweise Natriumhydrid, eingesetzt werden. Bevorzugt werden äquimolare Mengen der Base verwendet. Als Lösungsmittel sind zum Beispiel Dimethylformamid oder Dimethylsulfoxid, bevorzugt Dimethylformamid, geeignet.

Die Aufarbeitung und Isolierung erfolgt in üblicher Weise. Die erhaltene Verbindung kann gegebenenfalls in ihr physiologisch annehmbares Salz umgewandelt werden.

$b_1$) Zur Herstellung von Verbindungen der allgemeinen Formel I, in denen R für eine Gruppe der allgemeinen Formel III steht

$$-NH-R^2 \qquad (III)$$

und $R^2$ die oben angegebene Bedeutung besitzt, wird eine Verbindung der allgemeinen Formel Ia mit einem Hydrazinderivat, Hydroxylamin oder Guanidin in einem Lösungsmittel umgesetzt, wobei als Lösungsmittel ein alkoholisches Lösungsmittel, zum Beispiel Methanol, Ethanol oder Isopropanol, bevorzugt Ethanol, verwendet wird. Die Umsetzung erfolgt bei Raumtemperatur bis Rückflußtemperatur des Lösungsmittels, wobei letzteres bevorzugt wird.

Die Aufarbeitung und Isolierung erfolgt in üblicher Weise. Die erhaltene Verbindung kann gegebenenfalls in ihr physiologisch annehmbares Salz umgewandelt werden.

$b_2$) Zur Herstellung von Verbindungen der allgemeinen Formel I, bei denen R für eine Gruppe der allgemeinen Formel III

$$-NH-R^2 \qquad (III)$$

und $R^2$ die oben angegebene Bedeutung besitzt, wird eine Verbindung der allgemeinen Formel VIII

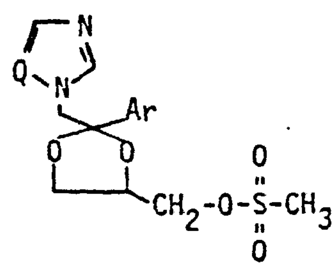

(VIII)

mit einer Verbindung der allgemeinen Formel X

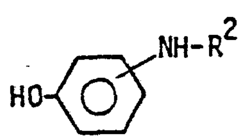

(X)

in der $R^2$ die oben angegebene Bedeutung besitzt, umgesetzt. Vorzugsweise werden äquimolare Mengen der Reaktanten eingesetzt. Die Umsetzung erfolgt bei den Bedingungen, wie sie weiter oben bezüglich der Verfahrensvariante $a_4$) angegeben wurden.

Die Aufarbeitung und Isolierung erfolgt in üblicher Weise. Die erhaltene Verbindung kann gegebenenfalls in ihr physiologisch annehmbares Salz umgewandelt werden.

$c_1$) Zur Herstellung von Verbindungen der allgemeinen Formel I, bei denen R für eine Gruppe der allgemeinen Formel IV

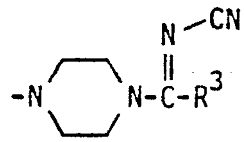

(IV)

steht und $R^3$ für eine Thiomethyl- oder Phenoxygruppe steht, wird eine Verbindung der allgemeinen Formel XI

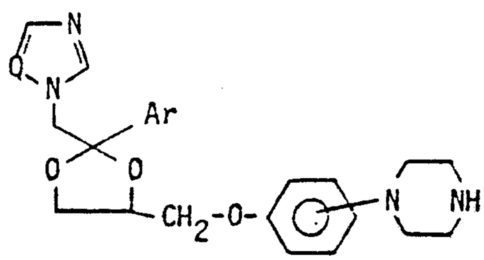

(XI)

mit einer Verbindung der allgemeinen Formel VII

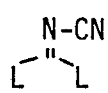

(VII)

in der L eine Thiomethyl- oder Phenoxygruppe als Austrittsgruppe bedeutet, in einem Lösungsmittel zu einer erfindungsgemäßen Verbindung der allgemeinen Formel Ib

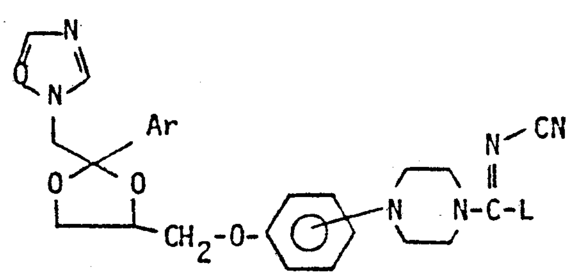

(Ib)

in der L eine Thiomethyl- oder Phenoxygruppe bedeutet, umgesetzt. Die als Ausgangsprodukt verwendete Verbindung XI kann in an sich bekannter Weise hergestellt werden (J. Med. Chem. 26, 611 (1983)).

Die Umsetzung erfolgt in einem inerten Lösungsmittel, wie Methanol, Ethanol oder Isopropanol, bevorzugt Isopropanol, und bei Raumtemperatur bis Rückflußtemperatur des verwendeten Lösungsmittels sowie unter Anwendung beispielsweise äquimolarer Mengen der einzelnen Reaktanten.

Die Aufarbeitung und Isolierung erfolgt in üblicher Weise. Die erhaltene Verbindung kann gegebenenfalls in ihr physiologisch annehmbares Salz umgewandelt werden.

$c_2$) Zur Herstellung von Verbindungen der allgemeinen Formel I, bei denen R für eine Gruppe der allgemeinen Formel IV

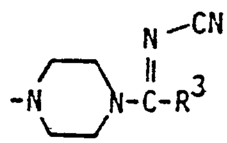

(IV)

steht und $R^3$ für eine unsubstituierte oder substituierte Aminogruppe steht, wird eine Verbindung der allgemeinen Formel Ib mit der entsprechend substituierten Aminoverbindung in einem Lösungsmittel umgesetzt. Als Lösungsmittel kommen Alkohole, wie Methanol, Ethanol oder Isopropanol, bevorzugt Isopropanol, in Betracht. Die Umsetzung erfolgt bei Raumtemperatur bis Rückflußtemperatur des verwendeten Lösungsmittels und unter Anwendung äquimolarer Mengen der Ausgangsverbindungen.

Die Aufarbeitung und Isolierung erfolgt in üblicher Weise. Die erhaltene Verbindung kann gegebenenfalls in ihr physiologisch annehmbares Salz umgewandelt werden.

$c_3$) Zur Herstellung von Verbindungen der allgemeinen Formel I, bei denen R für eine Gruppe der allgemeinen Formel IV und $R^3$ für eine Alkoxygruppe steht, wird eine Verbindung der allgemeinen Formel Ib mit einem Alkalialkoholat in dem entsprechenden Alkohol als Lösungsmittel umgesetzt. Als Alkalialkoholat wird vorzugsweise Natriumalkoholat verwendet. Die Umsetzung erfolgt bei Raumtemperatur bis Rückflußtemperatur des verwendeten Lösungsmittels und unter Anwendung äquimolarer Mengen der Ausgangsstoffe.

Die Aufarbeitung und Isolierung erfolgt in üblicher Weise. Die erhaltene Verbindung kann gegebenenfalls in ihr physiologisch annehmbares Salz umgewandelt werden.

$c_4$) Zur Herstellung von Verbindungen der allgemeinen Formel I, bei denen R für eine Gruppe der allgemeinen Formel IV steht und $R^3$ die oben angegebene Bedeutung besitzt, wird eine Verbindung der allgemeinen Formel VIII

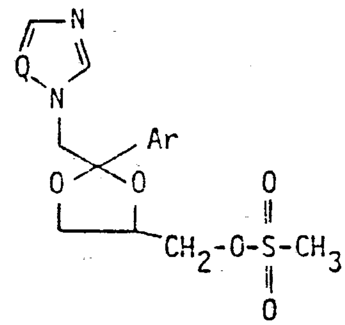

(VIII)

mit einer Verbindung der allgemeinen Formel XII

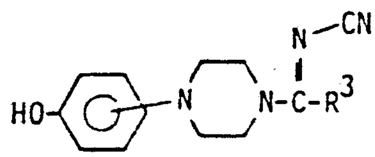

(XII)

in der $R^3$ die oben angegebene Bedeutung besitzt, umgesetzt. Die Umsetzung erfolgt basenkatalysiert in einem polaren aprotischen Lösungsmittel, wie Dimethylformamid oder Dimethylsulfoxid, bevorzugt Dimethylformamid. Als Basen können Natriumhydrid oder Kaliumcarbonat, vorzugsweise Natriumhydrid, eingesetzt werden, wobei die Anwendung von äquimolaren Mengen bevorzugt wird. Die Umsetzung erfolgt bei Raumtemperatur bis Rückflußtemperatur des verwendeten Lösungsmittels und vorzugsweise unter Anwendung äquimolarer Mengen der einzelnen Ausgangsstoffe.

Die Aufarbeitung und Isolierung erfolgt in üblicher Weise. Die erhaltene Verbindung kann gegebenenfalls in ihr physiologisch annehmbares Salz umgewandelt werden.

$d_1$) Zur Herstellung von Verbindungen der allgemeinen Formel I, bei denen R für eine Gruppe der allgemeinen Formel V

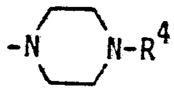

(V)

steht und $R^4$ die oben angegebene Bedeutung besitzt, wird eine Verbindung der allgemeinen Formel Ib mit einem Hydrazinderivat, Hydroxylamin oder Guanidin in einem Lösungsmittel umgesetzt. Als Lösungsmittel wird vorzugsweise ein Alkohol, wie Methanol, Ethanol oder Isopropanol, bevorzugt Ethanol, verwendet, und die Reaktionstemperatur ist Raumtemperatur bis Rückflußtemperatur des verwendeten Lösungsmittels, wobei letztere bevorzugt wird. Vorzugsweise wird unter Anwendung äquimolarer Mengen der einzelnen Reaktanten gearbeitet.

Die Aufarbeitung und Isolierung erfolgt in üblicher Weise. Die erhaltene Verbindung kann gegebenenfalls in ihr physiologisch annehmbares Salz umgewandelt werden.

$d_2$) Zur Herstellung von Verbindungen der allgemeinen Formel I, in denen R für eine Gruppe der allgemeinen Formel V steht und $R^4$ die oben angegebene Bedeutung besitzt, wird eine Verbindung der allgemeinen Formel VIII mit einer Verbindung der allgemeinen Formel XIII

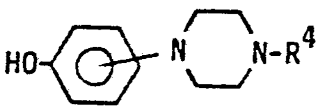

(XIII)

in der $R^4$ die oben angegebene Bedeutung besitzt, umgesetzt. Die Umsetzung erfolgt vorzugsweise bei den Bedingungen, wie sie oben bezüglich der Verfahrensvariante $d_1$) beschrieben wurden.

Die Aufarbeitung und Isolierung erfolgt in üblicher Weise. Die erhaltene Verbindung kann gegebenenfalls in ihr physiologisch annehmbares Salz umgewandelt werden.

Die in den Herstellungsstufen $a_1$) bis $d_2$) erhaltenen Verbindungen werden gegebenenfalls in an sich bekannter Weise in ihr physiologisch annehmbares Salz umgewandelt. Die Isolierung der erhaltenen Verbindung der allgemeinen Formel I erfolgt in üblicher Weise, beispielsweise durch Kristallisation oder Säulenchromatographie.

Mikrobiologische Untersuchungen

Eine Methode zur Bestimmung der mikrobiologischen Aktivität der erfindungsgemäßen Verbindungen gegenüber Hefen ist die Ermittlung der Hemmkonzentration 50% (HK 50%). Als HK 50% wird die Konzentration der Testsubstanz bezeichnet, die das Wachstum des Teststammes zu mehr als 50% im Vergleich zur unbehandelten Kontrolle gehemmt hat (vgl. J.N. Galgiani, D.A. Stevens; Antimicrob. Agents and Chemoth. 13, 249 - 254 (1978)).

Die mikrobiologische Aktivität wird am Beispiel 44 im Vergleich zu Ketoconazol gezeigt. Die übrigen erfindungsgemäßen Verbindungen zeigen ähnliche Aktivität.

Tabelle

| Stamm | pathogen | HK 50% µg/ml | |
|---|---|---|---|
| | | Beispiel 44 | Ketoconazol |
| Cand. pseudotropicalis | + | 0,00125 | 0,0125 |
| Cand. parapsilosis | + | 0,0125 | 0,0125 |
| Cand. krusei | + | 0,125 | 0,125 |
| Cand. curvata | + | 0,125 | 1,25 |
| Cand. albicans | + | 0,00125 | 0,0125 |
| Sacch. uvarum | (+) | 1,25 | 0,125 |
| Sacch. cerevisiae 1 | (+) | 1,25 | 1,25 |
| Sacch. fragilis | (+) | 0,0125 | 0,0125 |
| Sacch. cerevisiae 2 | (+) | 1,25 | 1,25 |
| Hansenula anomala | - | 0,00125 | 0,125 |
| Cryptococcus neoformans 1 | + | 0,0125 | 0,0125 |
| Cryptococcus neoformans 2 | + | 0,125 | 1,25 |
| Metschnikowia pulch | - | 0,00125 | 0,0125 |
| Sacch. rouxii | (+) | 0,00125 | 0,0125 |
| Geotrichum capitum | + | 0,125 | 1,25 |
| Rhodotorula spec. | - | 1,25 | 1,25 |

Die erfindungsgemäßen Verbindungen, vorzugsweise in der Form eines Salzes, können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmakologisch annehmbaren Trägern oder Verdünnungsmitteln hergestellt werden.

Die erfindungsgemäßen Verbindungen können daher für die orale, bukkale und parenterale Verabreichung formuliert werden, wobei die orale Verabreichung bevorzugt wird. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten. Alternativ kann der Wirkstoff auch in Pulverform zur Rekonstitution mit einem geeigneten Träger, zum Beispiel sterilem, pyrogenfreiem Wasser, vor dem Gebrauch vorliegen.

Für die orale Verabreichung ist eine geeignete Tagesdosis an erfindungsgemäßen Verbindungen 1 bis 4 Dosen, vorzugsweise 1 bis 2 Dosen, bis insgesamt 50 mg bis 500 mg, vorzugsweise 100 mg bis 250 mg, je nach Zustand des Patienten.
Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Beispiel 1

cis-N'-[4-[2-(2,4-Dichlorphenyl)-2-(1H-imidazol-1-ylmethyl)-1,3-dioxolan-4-ylmethoxy]-phenyl]-N''-cyano-phenylisoharnstoff

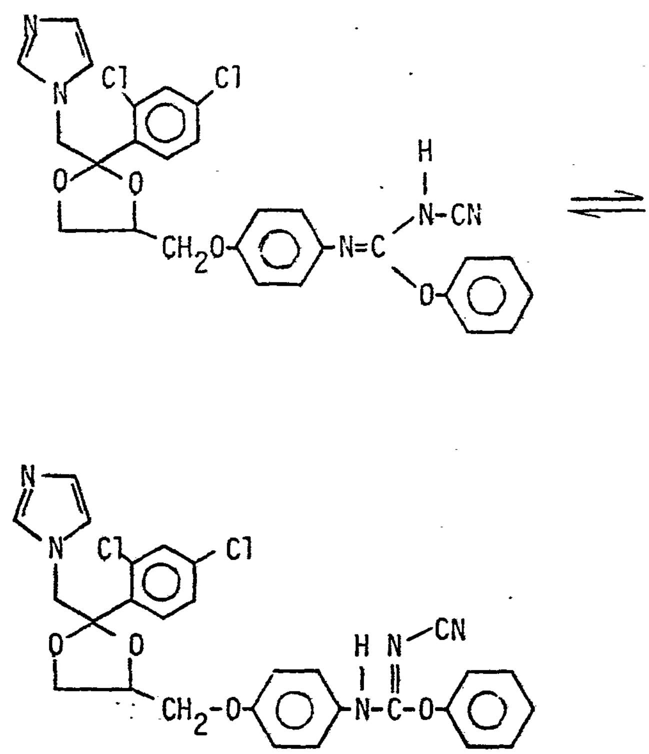

4,2 g (10 mmol) cis -4-[2-(2,4-Dichlorphenyl)-2-(1H-imidazol-1-ylmethyl)-1,3-dioxolan-4-ylmethoxy] benzolamin und 2.38 g (10 mmol) N-Cyanodiphenylimidocarbonat werden in 40 ml Isopropanol suspendiert und 1,5 h bei Raumtemperatur umgesetzt. Der anfallende Feststoff wird abgesaugt und aus Isopropanol umkristallisiert.

Farblose Kristalle vom Schmelzpunkt 91 - 93 °C

Rf = 0.60 (Tetrachlorkohlenstoff/Isopropanol 65 : 35 )
Ausbeute: 4.96 g (88 % d. Th.)

| | | | | |
|---|---|---|---|---|
| $C_{28}H_{23}Cl_2N_5O_4$ (564) | Ber.: | C 59.58 | H 4.11 | N 12.41 |
| | Gef.: | C 59.20 | H 4.43 | N 12.04 |

IR ($cm^{-1}$): 2200 ($\nu$-CN)

$^1$H-NMR-Spektrum: (CDCl$_3$, TMS als interner Standard)

$\delta$ = 3.10 - 3.47 (m) (C$\underline{H}$) 1 H,
3.57 - 3.93 (m) (C$\underline{H}_2$, C$\underline{H}$) 3 H,
4.17 - 4.53 (m) (C$\underline{H}_2$, C$\underline{H}$) 3 H
6.63 - 7.67 (m) (Aromaten-$\underline{H}$, N-$\underline{H}$) 16 H ppm.

Beispiel 2

cis-N'-[3-[2-(2,4-Dichlorphenyl)-2-(1H-imidazol-1-ylmethyl)-1,3-dioxolan-4-ylmethoxy]-phenyl]-N''-cyano-phenylisoharnstoff

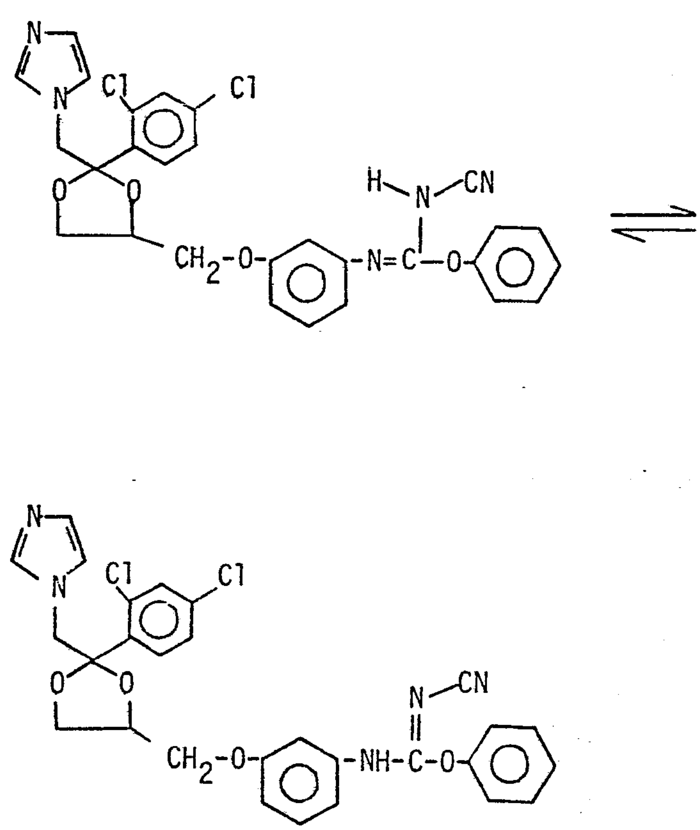

Die Herstellung erfolgt analog Beispiel 1.

Farblose Kristalle vom Schmelzpunkt 153 - 154 °C

Rf = 0.79 (Essigester / Methanol 80 : 20)

Ausbeute: 4.11 g (73 % d.Th.)

| | | | | |
|---|---|---|---|---|
| $C_{28}H_{23}Cl_2N_5O_4$ (564) | Ber.: | C 59.58 | H 4.11 | N 12.41 |
| | Gef.: | C 59.21 | H 4.10 | N 12.50 |

IR ($cm^{-1}$) : 2210 ($\nu$-CN)

$^1$H-NMR-Spektrum: (d$_6$-DMSO, TMS als interner Standard)

$\delta$ = 3.50 - 4.00 (m) (2 x CH, $CH_2$) 4 H,
4.20 - 4.60 (m) ($CH_2$, CH) 3 H,
6.67 - 7.73 (m) (Aromaten-H, N-H) 16 H ppm.

## Beispiel 3

cis-N'-[4-[2-(2,4-Dichlorphenyl)-2-(1H-1,2,4-triazol-1-ylmethyl)-1,3-dioxolan-4-ylmethoxy] phenyl]-N''-cyano-phenylisoharnstoff

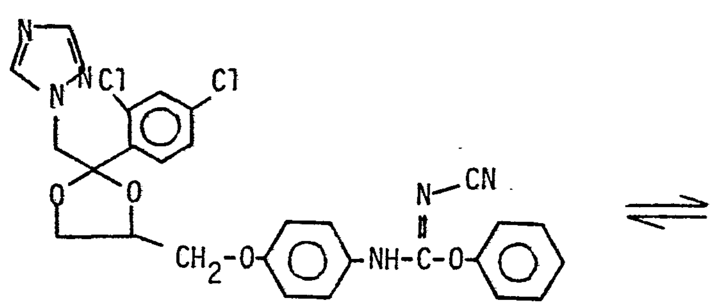

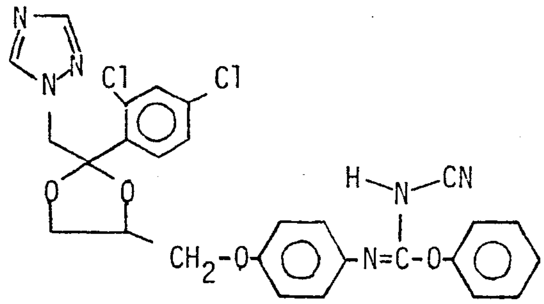

Die Herstellung erfolgt analog Beispiel 1.

Farblose Kristalle vom Schmelzpunkt 107 - 108 °C

Rf = 0.67 ($CHCl_3$ / EtOH 90 : 10)

Ausbeute: 4.52 g (82 % d.Th.)

$C_{27}H_{22}CL_2N_6O_4$ (565)

IR ($cm^{-1}$): 2215 ($\nu$-CN)

$^1$H-NMR-Spektrum: ($CDCl_3$, TMS als interner Standard)

$\delta$ = 3.33 - 4.10 (m) (2x$C\underline{H}_2$) 4 H,
4.20 - 4.47 (m) (C$\underline{H}$) 1 H,
4.73 (s, breit) ($C\underline{H}_2$) 2 H,
6.70 - 7.67 (m) (Aromaten-$\underline{H}$), 12 H,
7.87 (s, breit) (Aromaten-$\underline{H}$) 1 H,
8.17 (s, breit) (Aromaten-$\underline{H}$), 1 H,
9.10 (s, breit) (N-$\underline{H}$) (austauschbar mit $D_2O$) 1 H ppm.

Beispiel 4

cis-N'-[3-[2-(2,4-Dichlorphenyl)-2-(1H-1,2,4-triazol-1-ylmethyl)-1,3-dioxolan-4-ylmethoxy] phenyl]-N''-cyano-phenylisoharnstoff

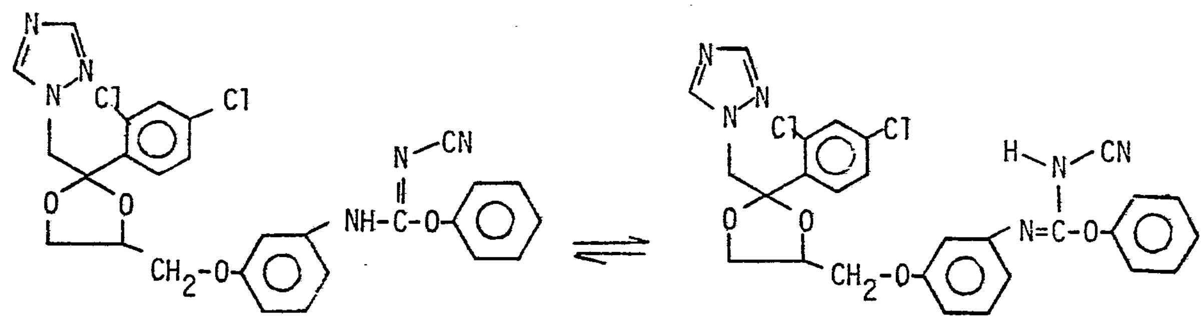

Die Herstellung erfolgt analog Beispiel 1.

Farblose Kristalle vom Schmelzpunkt 144 - 145 °C

Rf = 0.88 ($CHCl_3$ / EtOH 90 : 10)

Ausbeute: 4.24 g (75 % d.Th.)

$C_{27}H_{22}Cl_2N_6O_4$ (565)

IR ($cm^{-1}$) : 2205 ($\nu$-CN)

$^1$H-NMR-Spektrum: (d$_6$-DMSO, TMS als interner Standard)

$\delta$ = 3.57 - 4.03 (m) ($CH_2$, -$CH_2$) 4 H,
4.40 (m) (CH) 1 H,
4.83 (s) ($CH_2$) 2 H,
6.70 - 7.70 (m) (Aromaten-H) 12 H,
7.87 (s) (Aromaten-H) 1 H,
8.47 (s) (Aromaten-H) 1 H,
10.87 (s) (N-H) (austauschbar mit $D_2O$) 1 H ppm.

Beispiel 5

cis-N'-[4-[2-(2,4-Dichlorphenyl)-2-(1H-imidazol-1-ylmethyl)-1,3-dioxolan-4-ylmethoxy]-phenyl]-N''-cyano-methylisothioharnstoff

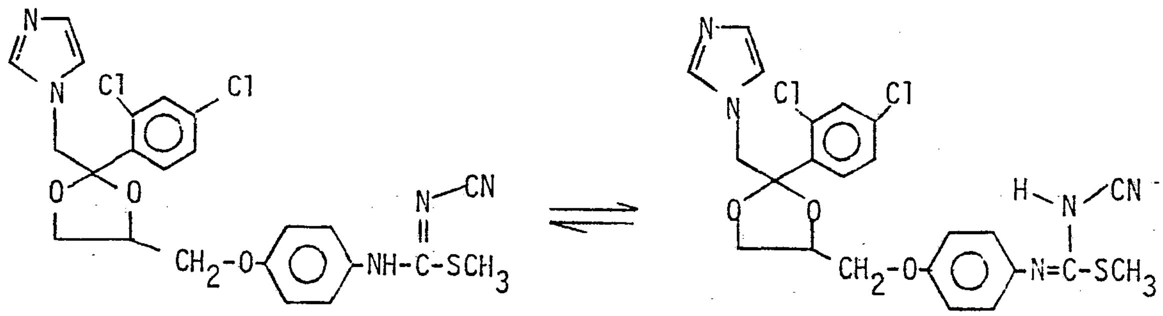

4.2 g (10 mmol) cis-4-[2-(2,4-Dichlorphenyl)-2-(1H-imidazol-1-ylmethyl)-1,3-dioxolan-4-ylmethoxy]benzolamin und 1.46 g (10 mmol) N-Cyanodimethyldithioimidocarbonat werden in 30 ml Isopropanol gelöst und 1 Stunde auf Rückflußtemperatur erhitzt. Die Reaktionslösung wird eingeengt und der Rückstand aus Diisopropylether umkristallisiert.

Farblose Kristalle vom Schmelzpunkt 90 - 92 $^{o}$ C (Zers.)

Rf = 0.61 ($CH_2Cl_2/CH_3OH$ 90 : 10 )

Ausbeute: 4.24 g (82 % d. Th.)

$C_{23}H_{21}Cl_2N_5O_3S$ (518) Ber.: C 53.29 H 4.08 N 13.51

Gef.: C 53.45 H 4.55 N 13.58

IR ($cm^{-1}$) : 2195 ($\nu$-CN)

$^1$H-NMR-Spektrum: (CDCl$_3$, TMS als interner Standard)

$\delta$ = 2.47 (s) ($SC\underline{H}_3$) 3 H,
3.13-3.40 (m) ( C$\underline{H}$) 1 H,
3.57 - 3.97 (m) (C$\underline{H}_2$, C$\underline{H}$) 3 H,
4.33 (m) (C$\underline{H}$) 1 H,

4.40 (s) ($CH_2$) 2 H,

6.63 - 7.67 (m) (Aromaten-H, N-H) 11 H ppm.

a) Herstellung von N''-Cyano-N'-(4-hydroxyphenyl)-methylisothioharnstoff

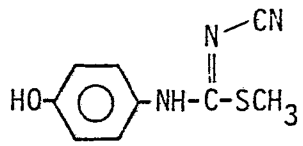

10.9 g (0.1 mol) 4-Hydroxyanilin werden mit 14.6 g (0.1 mol) Dimethyldithiocyanoimidocarbonat in 60 ml Methanol bei 40 °C 30 Minuten umgesetzt. Der ausgefallene Feststoff wird abgesaugt.

Farblose Kristalle vom Schmelzpunkt 153 - 154 °C

Ausbeute: 15.3 g (74 % d.Th.)

b) Herstellung von cis-N'-[4-[2-(2,4-Dichlorphenyl)-2-(1H-imidazol-1-yl-methyl)-1,3-dioxolan-4-ylmethoxy]-phenyl]-N''-cyano-methylisothioharnstoff.

0.425 g (14.8 mmol) Natriumhydrid werden in 5 ml wasserfreiem DMF vorgelegt und mit einer Lösung aus 2.8 g (14.8 mmol) N''-Cyano-N'-(4-hydroxyphenyl)-methylisothioharnstoff in 20 ml wasserfreiem DMF 1 Stunde bei Raumtemperatur umgesetzt. Anschließend tropft man eine Lösung aus 3.0 g (7.4 mmol) cis-2-(2,4-Dichlorphenyl)-2-(1H-imidazol-1-ylmethyl)-1,3-dioxolan-4-ylmethylmethansulfonat und 15 ml wasserfreiem DMF zum Reaktionsgemisch und erwärmt 8 Stunden auf 80 °C. Nach dem Abkühlen der Reaktionslösung engt man im Vakuum ein, nimmt den Rückstand in Dichlorethan auf, extrahiert die organische Phase mit einer 2,5 %-igen Natriumcarbonatlösung, stellt mit Wasser neutral und trocknet die organische Phase über $Na_2SO_4$. Nach dem Einengen der organischen Phase kristallisiert man den Rückstand aus Isopropanol und Diisopropylether um. Die physikalischen Daten stimmen mit denen von Beispiel 5 überein.

Beispiel 6

cis-N'-[3-[2-(2,4-Dichlorphenyl)-2-(1H-imidazol-1-ylmethyl)-1,3-dioxolan-4-ylmethoxy]-phenyl]-N''-cyano-methylisothioharnstoff

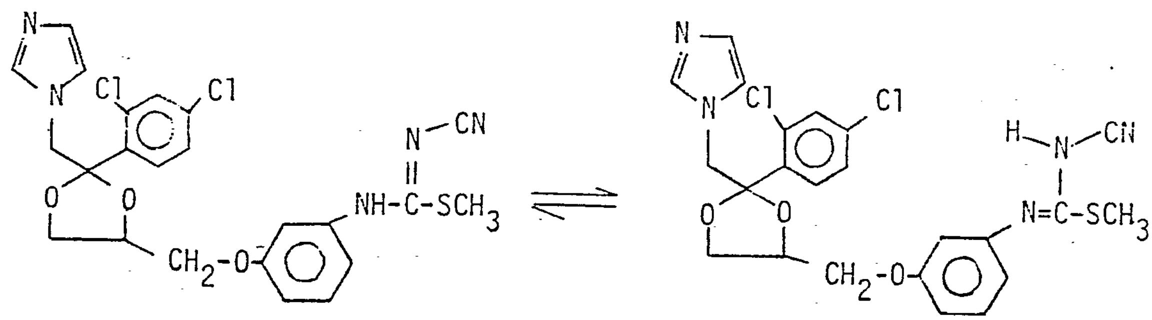

Die Herstellung erfolgt analog Beispiel 5.

Farblose Kristalle vom Schmelzpunkt 203 - 204 °C

Rf = 0.67 (Essigester/$CH_3OH$ 80 : 20)

Ausbeute: 3.26 g (63 % d.Th.)

$C_{23}H_{21}Cl_2N_5O_3S$ (518)

IR ($cm^{-1}$) : 2180 - 2175 ($\nu$-CN)

$^1$H-NMR-Spektrum: (d$_6$-DMSO, TMS als interner Standard)

$\delta$ = 2.70 (s) (S-C$\underline{H}_3$) 3 H,
3.53 - 4.00 (m) (2xC$\underline{H}_2$) 4 H,
4.40 (m) (C$\underline{H}$) 1 H,
4.57 (s) (C$\underline{H}_2$) 2 H,
6.73 - 7.73 (m)(Aromaten-$\underline{H}$, N-$\underline{H}$) 11 H ppm.

Beispiel 7

cis-N'-[4-[2-(2,4-Dichlorphenyl)-2-(1H-1,2,4-triazol-1-ylmethyl)-1,3-dioxolan-4-ylmethoxy] phenyl]-N''-cyano-methylisothioharnstoff

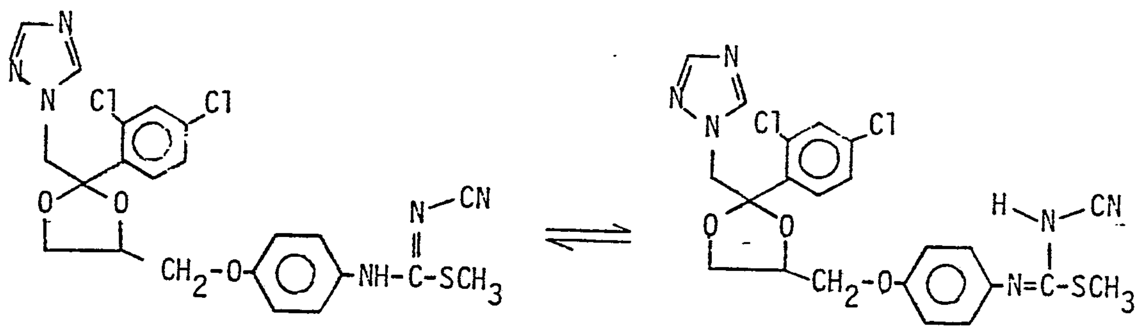

Die Herstellung erfolgt analog Beispiel 5.

Farblose Kristalle vom Schmelzpunkt 173 - 174 °C

Rf = 0.67 ($CHCl_3$ / EtOH 90 : 10)

Ausbeute: 4.31 g (83 % d.Th.)

$C_{22}H_{20}Cl_2N_6O_3S$ (519)

IR ($cm^{-1}$) : 2195 ( -CN)

$^1H$-NMR-Spektrum: (d$_6$-DMSO, TMS als interner Standard)

$\delta$ = 2.63 (s) ($SC\underline{H}_3$) 3 H,
3.63 - 4.03 (m) ($2xC\underline{H}_2$) 4 H,
4.38 (m) (C$\underline{H}$) 1 H,
4.83 (s) ($C\underline{H}_2$) 2 H,
6.87 - 7.73 (m) (Aromaten-$\underline{H}$) 7 H,
7.87 (s) (Aromaten-$\underline{H}$) 1 H,
8.43 (s) (Aromaten-$\underline{H}$) 1 H,
9.93 (breit) (N-$\underline{H}$) (austauschbar mit $D_2O$) 1 H ppm.

Beispiel 8

cis-N'-[3-[2-(2,4-Dichlorphenyl)-2-(1H-1,2,4-triazol-1-ylmethyl)-1,3-dioxolan-4-ylmethoxy]phenyl]-N''-cyano-methylisothioharnstoff

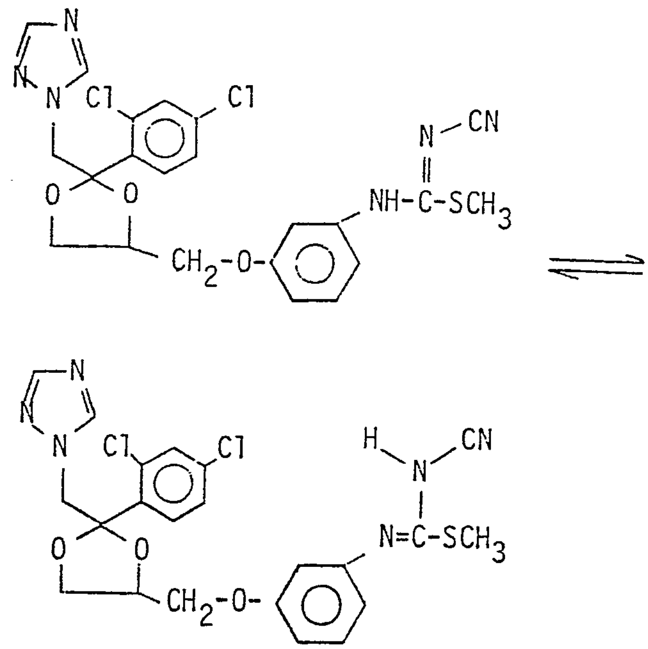

Die Herstellung erfolgt analog Beispiel 5.

Farblose Kristalle vom Schmelzpunkt 161 - 162 °C.

Rf = 0.70 ($CHCl_3$ / EtOH 90 : 10)

Ausbeute: 1.04 g (20 % d.Th.)

$C_{22}H_{20}Cl_2N_6O_3S$ (519)

IR ($cm^{-1}$): 2180 ($\nu$-CN)

$^1$H-NMR-Spektrum: ($CDCl_3$, TMS als interner Standard)

$\delta$ = 2.53 (s) (S-$C\underline{H}_3$) 3 H,
3.50 - 4.07 (m) (2x$C\underline{H}_2$) 4 H,
4.40 (m) (C$\underline{H}$) 1 H,
4.83 (s) ($C\underline{H}_2$) 2 H,
6.73 - 7.70 (m) (Aromaten-$\underline{H}$) 7 H,
7.90 (s) (Aromaten-$\underline{H}$) 1 H,
8.23 (s) (Aromaten-$\underline{H}$) 1 H,
9.00 (s) (N-$\underline{H}$) (austauschbar mit $D_2O$) 1 H ppm.

Beispiel 9

cis-N'[4-[2-(2,4-Dichlorphenyl)-2-(1H-imidazol-1-ylmethyl)-1,3-dioxolan-4-ylmethoxy]-phenyl]-N''-cyanomethylisoharnstoff

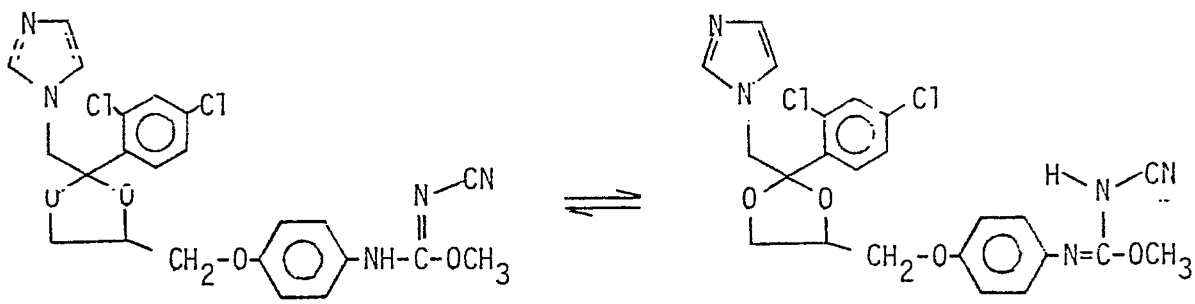

5.18 g (10 mmol) cis-N'-[4-[2-(2,4-Dichlorphenyl)-2-(1H-imidazol-1-ylmethyl)-1,3-dioxolan-4-ylmethoxy]-phenyl]-N''-cyano-methylisoharnstoff (Beispiel 5) wird mit 0,57 g (11 mmol) Natriummethylat in 50 ml Methanol 8 h bei Rückflußtemperatur umgesetzt. Nach dem Einengen des Lösungsmittels wird der Rückstand aus Diisopropylether umkristallisiert.

Farblose Kristalle vom Schmelzpunkt 96 - 97 °C

Rf = 0.39 (Diisopropylether/Methanol 70 : 30 )

Ausbeute: 3.36 g (67 % d.Th.)

| | | | | |
|---|---|---|---|---|
| $C_{23}H_{21}Cl_2N_5O_4$ (502) | Ber.: | C 54.99 | H 4.21 | N 13.94 |
| | Gef.: | C 54.76 | H 4.68 | N 13.73 |

IR ($cm^{-1}$) : 2200 ($\nu$-CN)

$^1$H-NMR-Spektrum: (d$_6$-DMSO, TMS als interner Standard)

$\delta$ = 3.30 (m) (C$\underline{H}$) 1 H,
3.50 - 3.93 (m) (C$\underline{H}_2$, C$\underline{H}$) 3 H,
3.77 (s) (OC$\underline{H}_3$) 3 H,
4.33 (m) (C$\underline{H}$) 1 H,
4.50 (s) (C$\underline{H}_2$) 2 H,
6.67 - 7.73 (m) (Aromaten-$\underline{H}$) 10 H,
10,0 (breit) (N-$\underline{H}$) (austauschbar mit $D_2O$) 1 H ppm.

a) Herstellung von N''-Cyano-N'-(4-hydroxyphenyl)-methylisoharnstoff

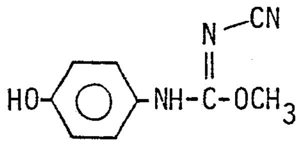

10.3 g (0.05 mol) N''-Cyano-N'-(4-hydroxyphenyl)-methylisothioharnstoff (siehe Beispiel 5a) werden in 50 ml Methanol gelöst und mit 18.2 ml (0.1 mol) 5,5 molarer Natriummethylatlösung versetzt. Anschließend wird 30 Minuten auf Rückflußtemperatur erhitzt, die Reaktionslösung nach dem Abkühlen mit konz. HCl-Lösung neutralisiert und im Vakuum eingeengt. Der verbleibende Rückstand wird in Essigsäureethylester aufgenommen, abfiltriert und die organische Lösung im Vakuum eingeengt. Der Rückstand wird in Methanol/Wasser 50 : 50 kristallisiert.

Farblose Kristalle vom Schmelzpunkt 174 - 175 °C

Ausbeute: 2.5 g (26.3 % d.Th.)

b) Herstellung von cis-N'-[4-[2-(2,4-Dichlorphenyl)-2-(1H-imidazol-1-ylmethyl)-1,3-dioxolan-4-ylmethoxy]-phenyl]-N''-cyanomethylisoharnstoff

Die Herstellung erfolgt analog Beispiel 5b.

Die physikalischen Daten der erhaltenen Verbindung stimmen mit denen von Beispiel 9 überein.

Beispiel 10

cis-N'-[3-[2-(2,4-Dichlorphenyl)-2-(1H-imidazol-1-ylmethyl)-1,3-dioxolan-4-ylmethoxy]-phenyl]-N''-cyano-methylisoharnstoff

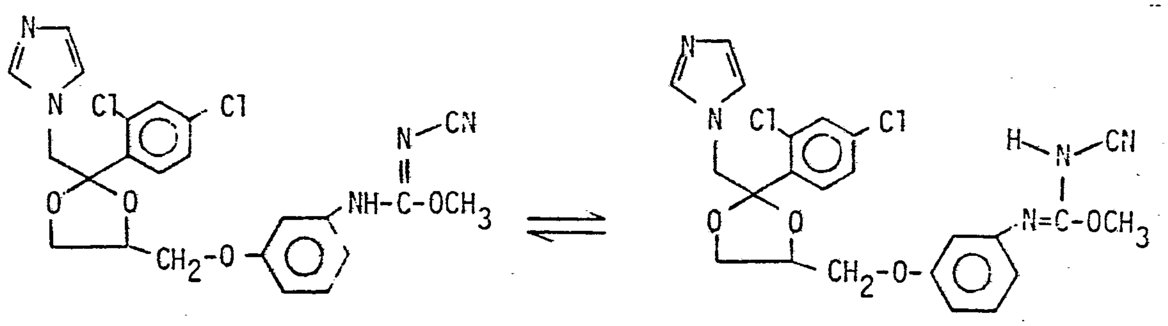

Die Herstellung erfolgt analog Beispiel 9.

Farblose Kristalle vom Schmelzpunkt 88 - 89 $^{o}$ C

Rf = 0.58 (Essigester / $CH_3OH$ 80 : 20)

Ausbeute: 3.77 g (75 % d.Th.)

| | | | | |
|---|---|---|---|---|
| $C_{23}H_{21}Cl_2N_5O_4$ (502) | Ber.: | C 54.99 | H 4.21 | N 13.93 |
| | Gef.: | C 54.68 | H 4.37 | N 13.90 |

IR ($cm^{-1}$): ($\nu$-CN)

$^{1}$H-NMR-Spektrum: (d$_6$-DMSO, TMS als interner Standard) $\delta$ = 3.47 - 4.00 (m) (2x$CH_3$) 4 H,
3.77 (s) ($OCH_3$) 3 H,
4.33 (m) (CH) 1 H,
4.50 (s) ($CH_2$) 2 H,
6.73 - 7.70 (m) (Aromaten-H) 10 H,
10.1 (breit) (N-H) (austauschbar mit $D_2O$) 1 H ppm.

0152596

Beispiel 11

cis-N-[4-[2-(2,4-Dichlorphenyl)-2-(1H-imidazol-1-ylmethyl)-1,3-dioxolan-4-ylmethoxy]-phenyl]-N''-cyano-ethylisoharnstoff

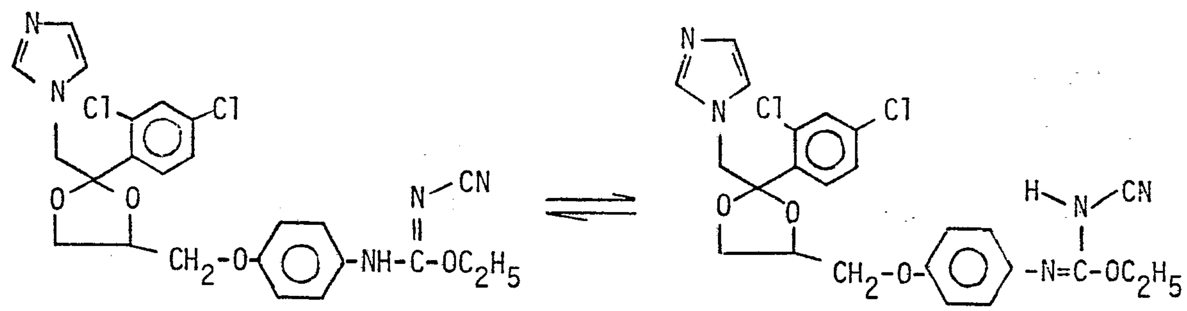

Die Herstellung erfolgt analog Beispiel 9 mit Natriumethylat.

Farblose Kristalle vom Schmelzpunkt 65 - 66 °C

Rf = 0.76 (Essigester / $CH_3OH$ 80 : 20)

Ausbeute: 2.17 g (42 % d.Th.)

$C_{24}H_{23}Cl_2N_5O_4$ (516)

IR ($cm^{-1}$) : 2200 ($\nu$-CN)

$^1$H-NMR-Spektrum: (CDCl$_3$, TMS als interner Standard)

$\delta$ = 1.30 (t) ($CH_3$) 3 H,
3.27 (dd) (CH) 1 H,
3.60 - 4.00 (m) ($CH_2$, CH) 3 H,
4.33 (q) ($CH_2$) 2 H,
4.43 (m) ($CH_2$, CH) 3 H,
6.67 - 7.70 (m) (Aromaten-H) 10 H,
8.33 (s, breit) (N-H) (austauschbar mit $D_2O$) 1 H ppm.

Beispiel 12

cis-N'-[4-[2-(2,4-Dichlorphenyl)-2-(1H-imidazol-1-ylmethyl)-1,3-dioxolan-4-ylmethoxy]-phenyl]-N''-cyano-propylisoharnstoff

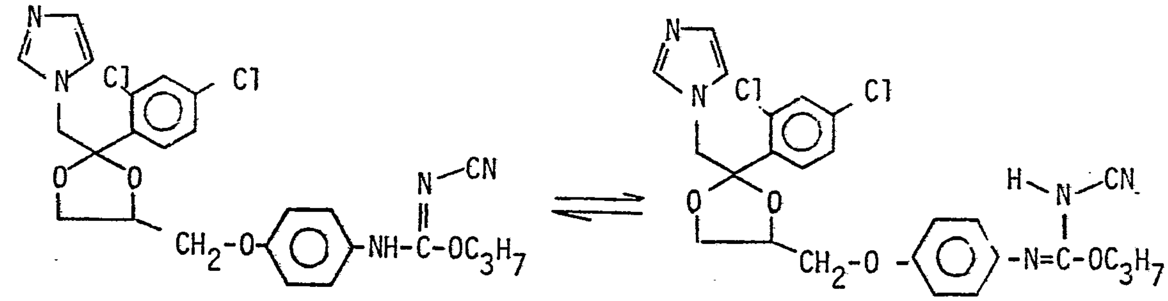

Die Herstellung erfolgt analog Beispiel 9 mit Natriumpropylat.

Farblose Kristalle vom Schmelzpunkt 138 - 140 °C

Rf = 0.66 (Essigester / Methanol 80 : 20)

Ausbeute: 2.39 g (45 % d.Th.)

$C_{25}H_{25}N_5O_4Cl_2$ (530)

IR ($cm^{-1}$) : 2200 ($\nu$ -CN)

$^1$H-NMR-Spektrum: (d$_6$-DMSO, TMS als interner Standard)

$\delta$ = 0.87 (t) ($CH_3$) 3 H,
1.60 (m) ($CH_2$) 2 H,
3.70 (m) (2x$CH_2$) 4 H,
4.17 (t) ($OCH_2$) 2 H,
4.37 (m) (CH) 1 H,
4.53 (s) ($CH_2$) 2 H,
6.77 - 7.73 (m) (Aromaten-H) 10 H,
10.1 (s) (N-H) (austauschbar mit $D_2O$) 1 H ppm.

Beispiel 13

cis-N'-[4-[2-(2,4-Dichlorphenyl)-2-(1H-imidazol-1-ylmethyl)-1,3-dioxolan-4-ylmethoxy]-phenyl]-N''-cyano-isopropylisoharnstoff

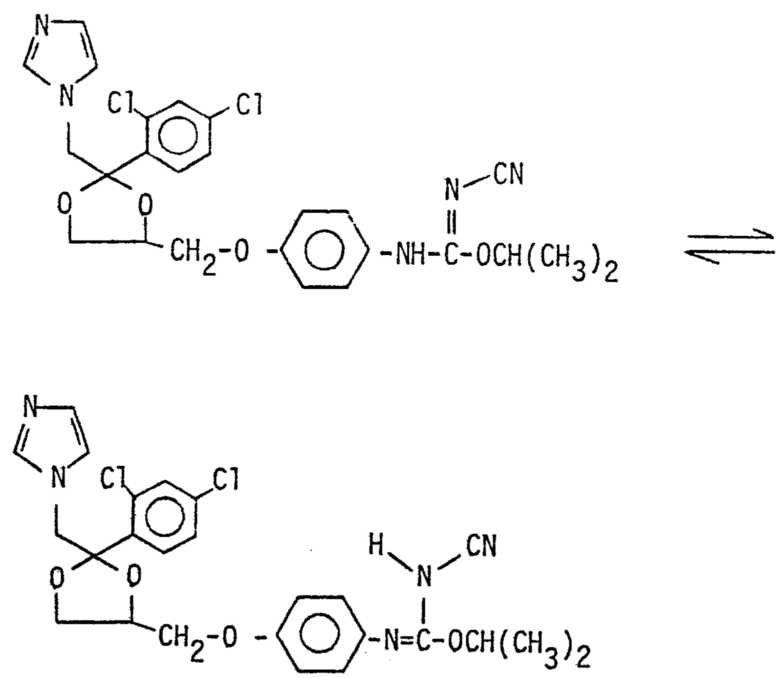

Die Herstellung erfolgt analog Beispiel 9 mit Natriumisopropylat.

Farblose Kristalle vom Schmelzpunkt 136 - 137 °C

Rf = 0.66 (Essigester / Methanol 80 : 20)

Ausbeute: 3.02 g (57 % d.Th.)

$C_{25}H_{25}N_5O_4Cl_2$ (530)

IR ($cm^{-1}$) : 2200 ($\nu$-CN)

$^1$H-NMR-Spektrum: (d$_6$-DMSO, TMS als interner Standard)

$\delta$ = 1.57 (d) (2x$CH_3$) 6 H,
3.50 - 4.00 (m) (2x$CH_2$) 4 H,
4.38 (m) (CH) 1 H,
4.57 (s) ($CH_2$) 2 H,

5.07 (h) (C$\underline{H}$) 1 H,

6.70 - 7.73 (m) (Aromaten-$\underline{H}$) 10 H,

10.3 (s) (N-$\underline{H}$) (austauschbar mit $D_2O$) 1 H ppm.

Beispiel 14

cis-N'-[4-[2-(2,4-Dichlorphenyl)-2-(1H-imidazol-1-ylmethyl)-1,3-dioxolan-4-ylmethoxy]-phenyl]-N''-cyano-n-butylisoharnstoff

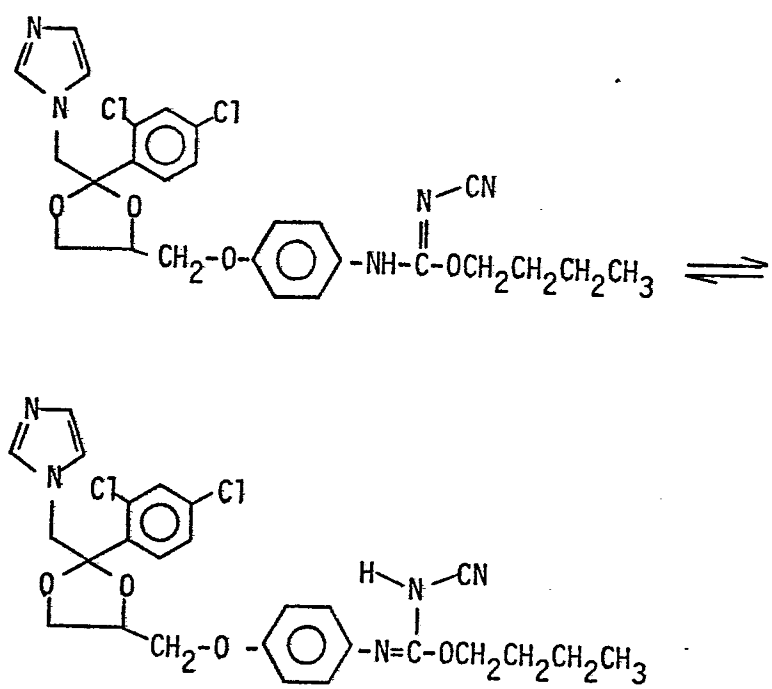

Die Herstellung erfolgt analog Beispiel 9 mit Natriumbutylat.

Farblose Kristalle vom Schmelzpunkt 136 - 137 °C

Rf = 0.67 (Essigester / Methanol 80 : 20)

Ausbeute: 2.02 g (37 % d.Th.)

$C_{26}H_{27}N_5O_4Cl_2$ (544)

IR ($cm^{-1}$) : 2200 ($\nu$-CN)

$^1$H-NMR-Daten:
($d_6$-DMSO, TMS als interner Standard)

$\delta$ = 0.87 (t) ($CH_3$) 3 H,
1.13 - 1.80 (m) (2x$CH_2$) 4 H,
3.50 - 4.00 (m) (2x$CH_2$) 4 H,
4.17 (t) (O-$CH_2$) 2 H,
4.33 (m) (CH) 1 H,
4.50 (s) ($CH_2$) 2 H,
6.73 - 7.73 (m) (Aromaten-H) 10 H,
10.1 (s) (N-H) (austauschbar mit $D_2O$) 1 H ppm.

## Beispiel 15

cis-N'-[4-[2-(2,4-Dichlorphenyl)-2-(1H-imidazol-1-ylmethyl)-1,3-dioxolan-4-ylmethoxy]-phenyl]-N''-cyano-2-methoxyethylisoharnstoff

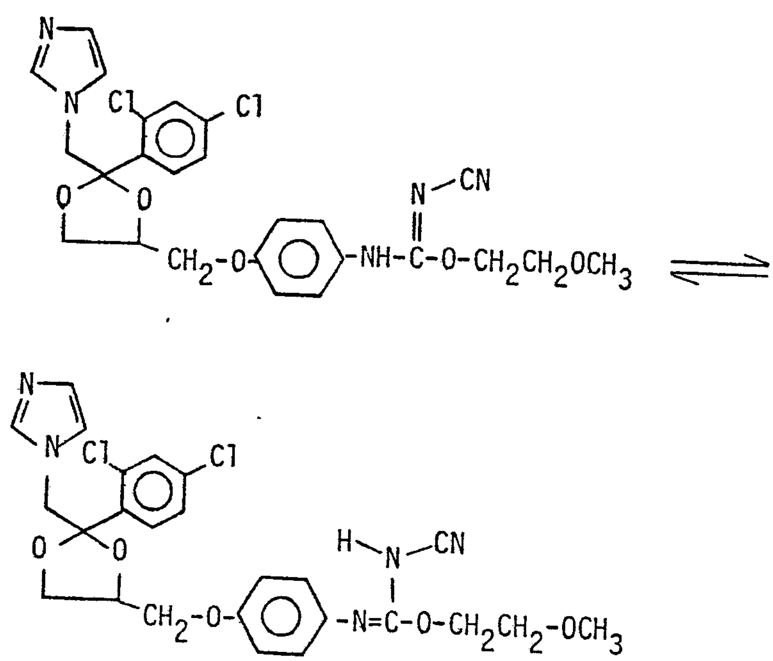

Die Herstellung erfolgt analog Beispiel 9 mit Natriummethoxyethylat.

Farblose Kristalle vom Schmelzpunkt 131 - 132 °C

Rf = 0.57 (Essigester / Methanol 80 : 20)

Ausbeute: 3.66 g (67 % d.Th.)

$C_{25}H_{25}N_5O_5Cl_2$ (546)

IR ($cm^{-1}$) : 2200 ($\nu$-CN)

$^1$H-NMR-Daten: (d$_6$-DMSO, TMS als interner Standard)

$\delta$ = 3.23 (s) ($OC\underline{H}_3$) 3 H,
3.40 - 4.00 (m) ($3xC\underline{H}_2$) 6 H,
4.17 - 4.47 (m) ($C\underline{H}$, $C\underline{H}_2$) 3 H,
4.53 (s) ($C\underline{H}_2$) 2 H,
6.67 - 7.73 (m) (Aromaten-$\underline{H}$) 10 H,
10.2 (s) (N-$\underline{H}$) (austauschbar mit $D_2O$) 1 H ppm.

0152596

Beispiel 16

cis-N'-[4-[2-(2,4-Dichlorphenyl)-2-(1H-imidazol-1-ylmethyl)-1,3-dioxolan-4-ylmethoxy]-phenyl]-N''-cyano-N'''-methylguanidin

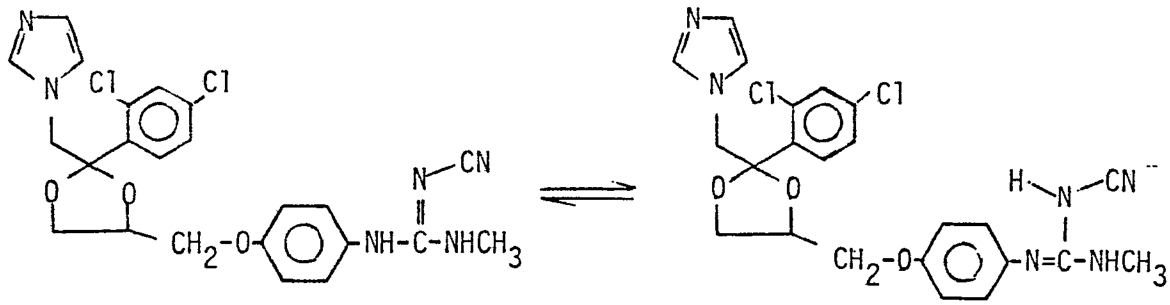

5.65 g (10 mmol) cis N'-[4-[2-(2,4-Dichlorphenyl)-2-(1H-imidazol-1-ylmethyl)-1,3-dioxolan-4-ylmethoxy]-phenyl]-N''-cyano-phenylisoharnstoff (Beispiel 1) werden in 100 ml Ethanol gelöst und mit 0.47 (15 mmol) Methylamin 3 h bei Raumtemperatur umgesetzt. Die Reaktionslösung wird im Vakuum eingeengt und der Rückstand aus Isopropanol umkristallisiert.

Farblose Kristalle vom Schmelzpunkt 187 - 189 $^{o}$ C

Rf = 0.52 ($CH_2Cl_2$ / iPrOH 80 : 20)

Ausbeute: 3.26 g (65 % d.Th.)

$C_{23}H_{22}Cl_2N_6O_3$ (501)

IR ($cm^{-1}$) : 2180 ($\nu$-CN)

$^1$H-NMR-Spektrum: (d$_6$-DMSO, TMS als interner Standard)

$\delta$ = 2.75 (d) (N-C$\underline{H}_3$) 3 H,
3.13 - 3.47 (m) (C$\underline{H}$) 1 H,
3.53 - 4.00 (m) (C$\underline{H}_2$, C$\underline{H}$) 3 H,
4.37 (m) (C$\underline{H}$) 1 H,

4.53 (s) ($C\underline{H}_2$) 2 H,

6.87 - 7.80 (m) (Aromaten-H, N-H) 11 H,

8.77 (s) (N-H) (austauschbar mit $D_2O$) 1 H ppm.

Beispiel 17

cis-N'-[3-[2-(2,4-Dichlorphenyl)-2-(1H-imidazol-1-ylmethyl)-1,3-dioxolan-4-ylmethoxy]-phenyl]-N''-cyano-N'''-methylguanidin

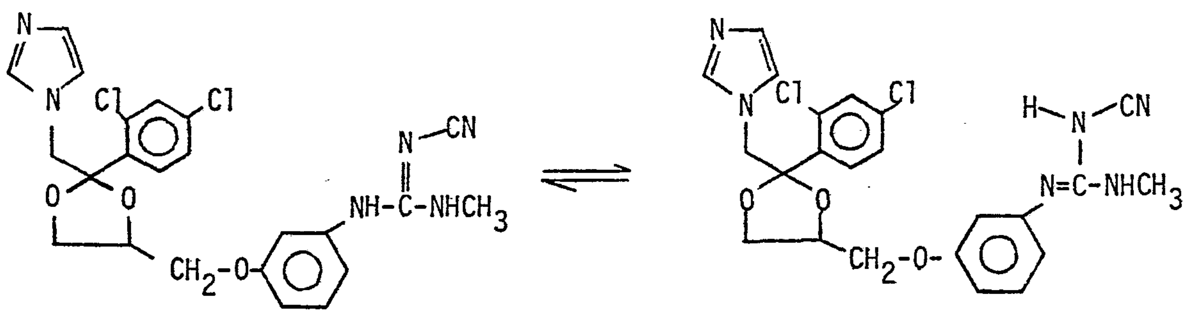

Die Herstellung erfolgt analog Beispiel 16.

Farblose Kristalle vom Schmelzpunkt 87 - 88 °C

Rf = 0.51 (Essigester / $CH_3OH$ 80 : 20 )

Ausbeute: 3.00 g (60 % d.Th.)

$C_{23}H_{22}Cl_2N_6O_3$ (501)

IR ($cm^{-1}$) : 2180 ($\nu$-CN)

$^1$H-NMR-Spektrum:
($d_6$-DMSO, TMS als
interner Standard)

$\delta$ = 2.78 (d) (N-C$\underline{H}_2$) 3 H,
3.50 - 4.00 (m) (2xC$\underline{H}_2$) 4 H,
4.33 (m) (C$\underline{H}$) 1 H,
4.50 (s) (C$\underline{H}_2$) 2 H,
6.53 - 7.70 (m) (Aromaten-$\underline{H}$, N-$\underline{H}$) 11 H,
8.93 (s) (N-H) (austauschbar mit $D_2O$) 1 H ppm.

Beispiel 18

cis-N'-[3-[2-(2,4-Dichlorphenyl)-2-(1H-1,2,4-triazol-1-ylmethyl)-1,3-dioxolan-4-ylmethoxy]-phenyl]-N''-cyano-N'''-methylguanidin

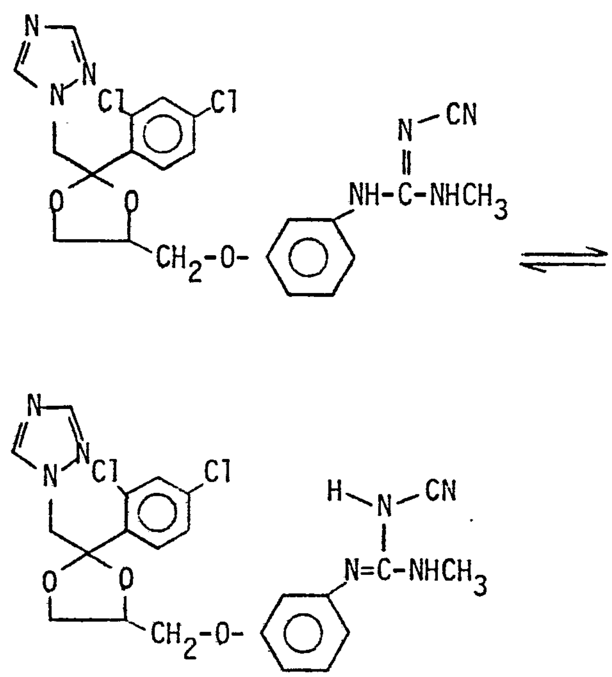

Die Herstellung erfolgt analog Beispiel 16.

Farblose Kristalle vom Schmelzpunkt 147 - 148 °C

Rf = 0.60 ($CHCl_3$/Ethanol 90 : 10)

Ausbeute: 2.36 g (47 % d.Th.)

$C_{22}H_{21}Cl_2N_7O_3$ (502)

IR ($cm^{-1}$): 2180 ( -CN)

$^1$H-NMR-Spektrum: ($CDCl_3$, TMS als interner Standard)

= 2.95 (d) ($C\underline{H}_3$) 3 H,
3.53 - 4.00 (m) ($2xC\underline{H}_2$) 4 H,
4.40 (m) (C$\underline{H}$) 1 H,
4.80 (s) ($C\underline{H}_2$) 2 H,
6.23 (q, breit) (N-$\underline{H}$) (austauschbar mit $D_2O$) 1 H,
6.63 - 8.23 (m) (Aromaten-$\underline{H}$) 9 H,
8.00 (s) (N-$\underline{H}$) (austauschbar mit $D_2O$) ppm.

## Beispiel 19

cis-N'-[4-[2-(2,4-Dichlorphenyl)-2-(1H-imidazol-1-ylmethyl)-1,3-dioxolan-4-ylmethoxy]-phenyl]-N''-cyano-guanidin

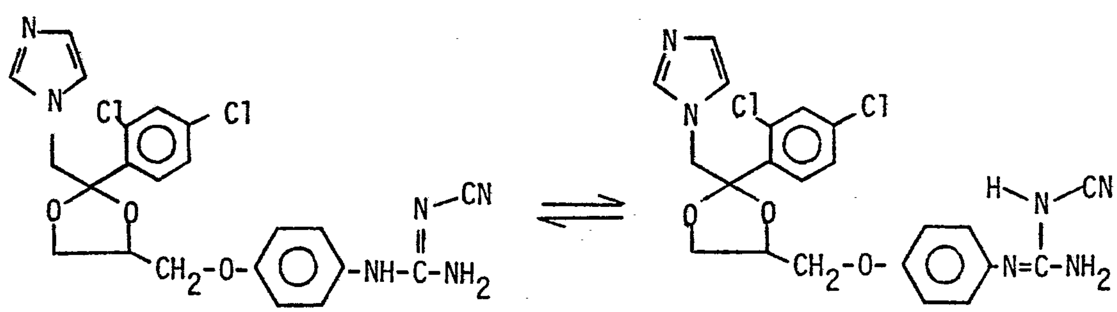

Die Herstellung erfolgt analog Beispiel 16 mit ethanolischer Ammoniaklösung.

Hellgelbe Kristalle vom Schmelzpunkt 98 - 100 °C

Rf = 0.31 (Essigester / $CH_3OH$ 80 : 20)

Ausbeute: 2.78 g (57 % d.Th.)

$C_{22}H_{20}Cl_2N_6O_3$ (487)

Ber.: C 54.22 H 4.14 N 17.24

Gef.: C 54.27 H 4.13 N 16.82

IR ($cm^{-1}$) : 2190 ($\nu$ -CN)

$^1$H-NMR-Spektrum: (CDCl$_3$, TMS als interner Standard)

$\delta$ = 3.13 - 3.93 (m) (2x$CH_2$) 4 H,
4.27 (m) (CH) 1 H,
4.43 (s) ($CH_2$) 2 H,
6.27 (m, breit) ($NH_2$) (austauschbar mit $D_2O$) 2 H,
6.57 - 7.77 (m) (Aromaten-H) 10 H,
8.67 (breit) (N-H) (austauschbar mit $D_2O$) 1 H ppm.

## Beispiel 20

cis-N'-[4-[2-(2,4-Dichlorphenyl)-2-(1H-imidazol-1-ylmethyl)-1,3-dioxolan-4-ylmethoxy]-phenyl]-N''-cyano-N'''-ethylguanidin

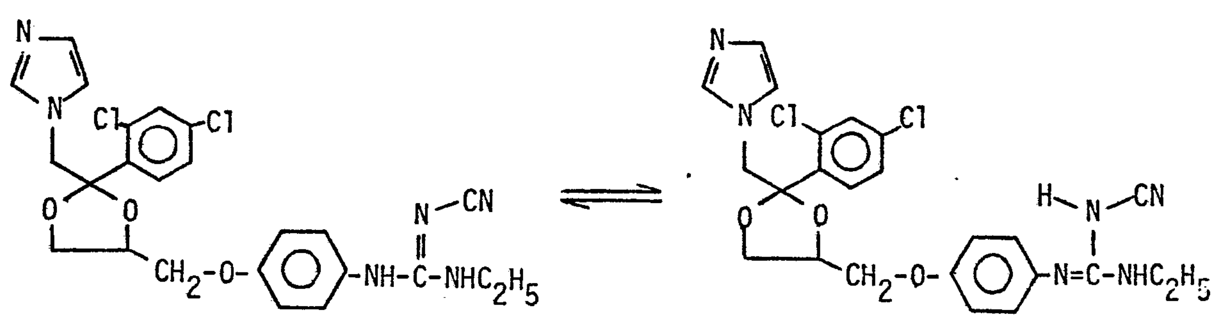

Die Herstellung erfolgt analog Beispiel 16 mit Ethylamin.

Farblose Kristalle vom Schmelzpunkt 180 - 181 °C

Rf = 0.55 (Essigester / $CH_3OH$ 80 : 20)

Ausbeute: 3.86 g (75 % d.Th.)

$C_{24}H_{24}Cl_2N_6O_3$ (515)

| | | | |
|---|---|---|---|
| Ber.: | C 55.93 | H 4.69 | N 16.30 |
| Gef.: | C 55.56 | H 4.81 | N 16.06 |

IR ($cm^{-1}$): 2180 ($\nu$-CN)

$^1$H-NMR-Spektrum: ($d_6$-DMSO, TMS als interner Standard)

$\delta$ = 1.07 (t) ($-C\underline{H}_3$) 3 H,
3.27 (m) ($C\underline{H}_2$) 2 H,
3.53 - 4.07 (m) ($2xC\underline{H}_2$) 4 H,
4.40 (m) ($C\underline{H}$) 1 H,
4.57 (s) ($C\underline{H}_2$) 2 H,
6.70 - 7.80 (m) (Aromaten-$\underline{H}$, $N\underline{H}$) 11 H,
8.77 (s) ($N-\underline{H}$) (austauschbar mit $D_2O$) 1 H ppm.

Beispiel 21

cis-N'-[4-[2-(2,4-Dichlorphenyl)-2-(1H-imidazol-1-ylmethyl)-1,3-dioxolan-4-ylmethoxy]-phenyl]-N''-cyano-N'''-isopropylguanidin

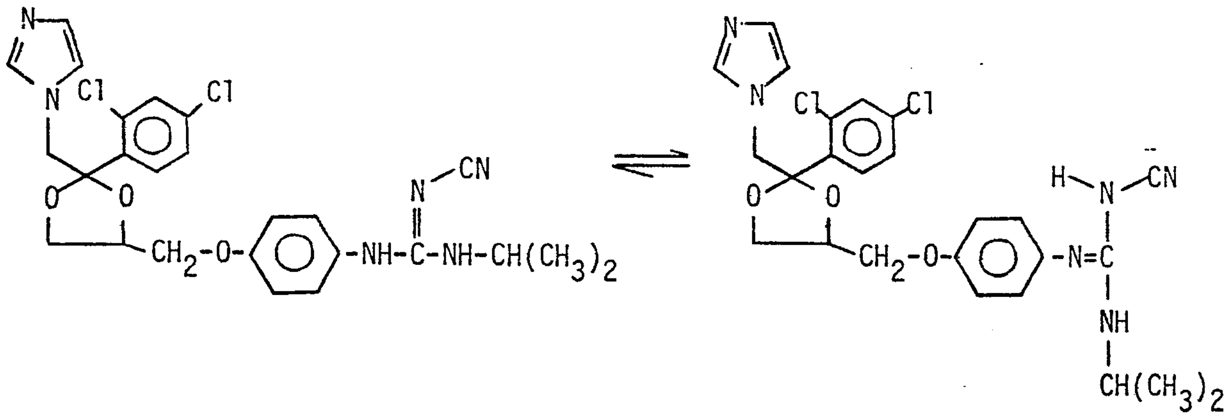

Die Herstellung erfolgt analog Beispiel 16 mit Isopropylamin.

Farblose Kristalle vom Schmelzpunkt 88 - 90 °C.

Rf = 0.56 (Essigester / Methanol 80 : 20 )

Ausbeute: 3.76 g (71 % d.Th.)

$C_{25}H_{26}N_6O_3Cl_2$ (529)

IR ($cm^{-1}$) : 2180 ($\nu$-CN)

$^1$H-NMR-Spektrum: (d$_6$-DMSO, TMS als interner Standard)

$\delta$ = 1.13 (d) (2x$CH_3$) 6 H,
3.47 - 4.17 (2x$CH_2$, CH) 5 H,
4.40 (m) (CH) 1 H,
4.60 (s) ($CH_2$) 2 H,
6.53 - 7.77 (m) (Aromaten-H, N-H) 11 H,
8.80 (s) (N-H) (austauschbar mit $D_2O$) 1 H ppm.

Beispiel 22

cis-N'-[3-[2-(2,4-Dichlorphenyl)-2-(1H-1,2,4-triazol-1-ylmethyl)-1,3-dioxolan-4-ylmethoxy]-phenyl]-N''-cyano-N'''-propylguanidin

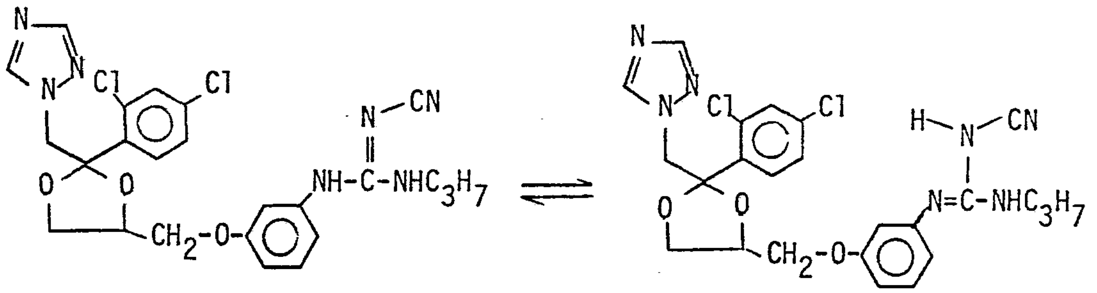

Die Herstellung erfolgt analog Beispiel 16.

Farblose Kristalle vom Schmelzpunkt 146 - 147 °C

Rf = 0.62 (Chloroform/Ethanol 90 : 10)

$C_{24}H_{25}Cl_2N_7O_3$ (530) Ber.: C 54.34 H 4.75

Gef.: C 54.68 H 4.79

IR ($cm^{-1}$) : 2185 ($\nu$-CN)

$^1$H-NMR-Spektrum: (CDCl$_3$, TMS als interner Standard)

$\delta$ = 0.88 (t) ($CH_3$) 3 H,
1.57 (m) ($CH_2$) 2 H,
3.27 (m) ($CH_2$) 2 H,
3.73 (d) ($CH_2$) 2 H,
3.87 (m) (2x$CH_2$) 4 H,
4.40 (m) (CH) 1 H,
4.77 (s) ($CH_2$) 2 H,
5.87 (t) (N-H) (austauschbar mit $D_2O$) 1 H,
6.63 - 7.73 (m) (Aromaten-H) 7 H,
7.77 (s) (Aromaten-H) 1 H,
8.00 (s) (N-H) (austauschbar mit $D_2O$) 1 H,
8.17 (s) (Aromaten-H) 1 H ppm.

Beispiel 23

cis-N'-[4-[2-(2,4-Dichlorphenyl)-2-(1H-imidazol-1-ylmethyl)-1,3-dioxolan-4-ylmethoxy]-phenyl]-N''-cyano-N'''-propylguanidin

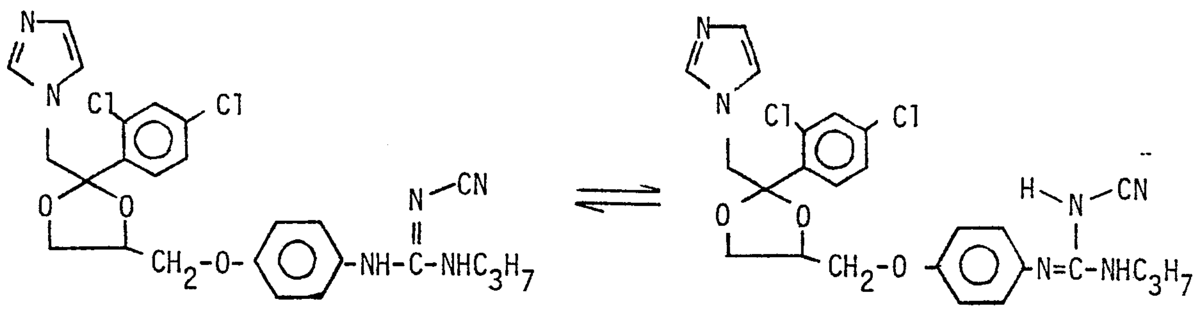

Die Herstellung erfolgt analog Beispiel 16 mit Propylamin.

Farblose Kristalle vom Schmelzpunkt 91 - 92 °C

Rf = 0.58 (Essigester / $CH_3OH$ 80 : 20 )

Ausbeute: 3.76 g (71 % d.Th.)

$C_{25}H_{26}Cl_2N_6O_3$ (529)

IR ($cm^{-1}$): 2180 ($\nu$-CN)

$^1$H-NMR-Spektrum: (d$_6$-DMSO, TMS als interner Standard)

$\delta$ = 0.83 (t) ($CH_3$) 3 H,
1.50 (m) ($CH_2$) 2 H,
3.13 (m) ($CH_2$) 2 H,
3.50 - 4.00 (m) (2x$CH_2$) 4 H,
4.37 (m) (CH) 1 H,
4.50 (s) ($CH_2$) 2 H,
6.70 - 7.70 (m) (Aromaten-H, N-H) 11 H,

8.73 (s) (N-H) (austauschbar mit $D_2O$)
1 H ppm.

Beispiel 24

cis-N'-[4-[2-(2,4-Dichlorphenyl)-2-(1H-imidazol-1-ylmethyl)-1,3-dioxolan-4-ylmethoxy]-phenyl]-N''-Cyano-N'''-cyclohexylguanidin

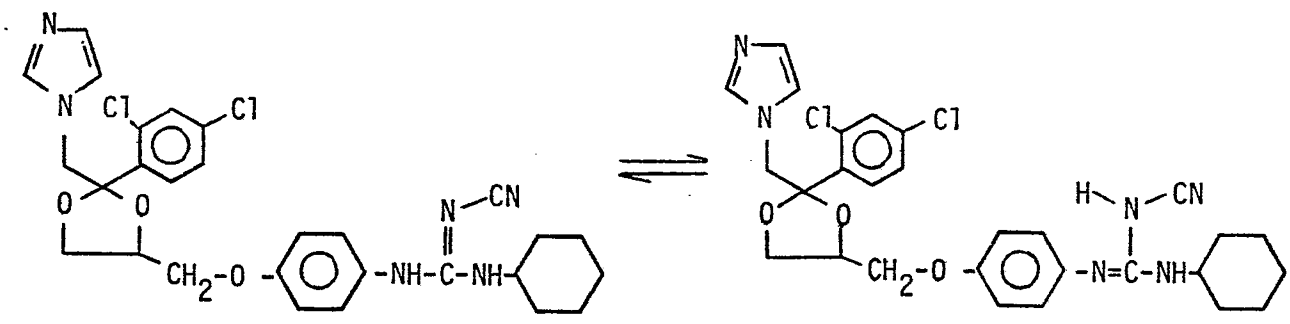

Die Herstellung erfolgt analog Beispiel 16 mit Cyclohexylamin.

Farblose Kristalle vom Schmelzpunkt 94 - 95 °C

Rf = 0.62 (Essigester / $CH_3OH$ 80 : 20)

Ausbeute: 3.73 g (67 % d.Th.)

$C_{27}H_{29}Cl_2N_6O_3$ (556)

IR ($cm^{-1}$): 2180 ($\nu$-CN)

$^1$H-NMR-Spektrum: (d$_6$-DMSO, TMS als interner Standard)

δ = 1.07 - 1.97 (m) (4x$CH_2$, CH) 11 H,
3.27 - 4.00 (m) (2x$CH_2$) 4 H,
4.37 (m) (CH) 1 H,
4.53 (s) ($CH_2$) 2 H,
6.53 - 7.73 (m) (Aromaten-H, N-H) 11 H,
8.67 (breit) (N-H) (austauschbar mit $D_2O$) 1 H ppm.

Beispiel 25

cis-N'-[3-[2-(2,4-Dichlorphenyl)-2-(1H-imidazol-1-ylmethyl)-1,3-dioxolan-4-ylmethoxy]-phenyl]-N''-cyano-N'''-dimethylguanidin

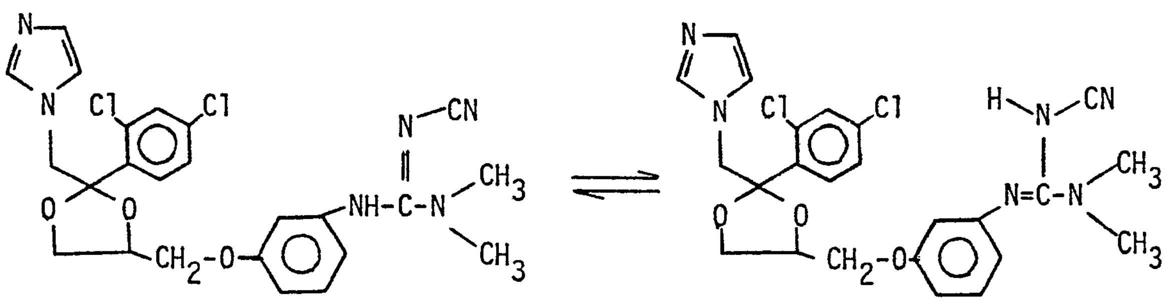

Die Herstellung erfolgt analog Beispiel 16 mit Dimethylamin.

Farblose Kristalle vom Schmelzpunkt 55 - 56 °C

Rf = 0.22 (Essigester / $CH_3OH$ 80 : 20)

Ausbeute: 2.42 g (47 % d. Th.)

$C_{24}H_{24}Cl_2N_6O_3$ (515) x $H_2O$

IR ($cm^{-1}$): 2180 ($\nu$-CN)

$^1$H-NMR-Spektrum: (d$_6$-DMSO, TMS als interner Standard)

$\delta$ = 3.00 (s) ($N(C\underline{H}_3)_2$) 6 H,
3.50 - 4.00 (m) (2x$C\underline{H}_2$) 4 H,
4.37 (m) (C$\underline{H}$) 1 H,
4.53 (s) ($C\underline{H}_2$) 2 H,
6.47 - 7.73 (m) (Aromaten-$\underline{H}$) 10 H,
9.17 (s) (N-$\underline{H}$) (austauschbar mit $D_2O$) 1 H ppm.

## Beispiel 26

cis-N'-[4-[2-(2,4-Dichlorphenyl)-2-(1H-imidazol-1-ylmethyl)-1,3-dioxolan-4-ylmethoxy]-phenyl]-N''-cyano-N'''-phenylguanidin

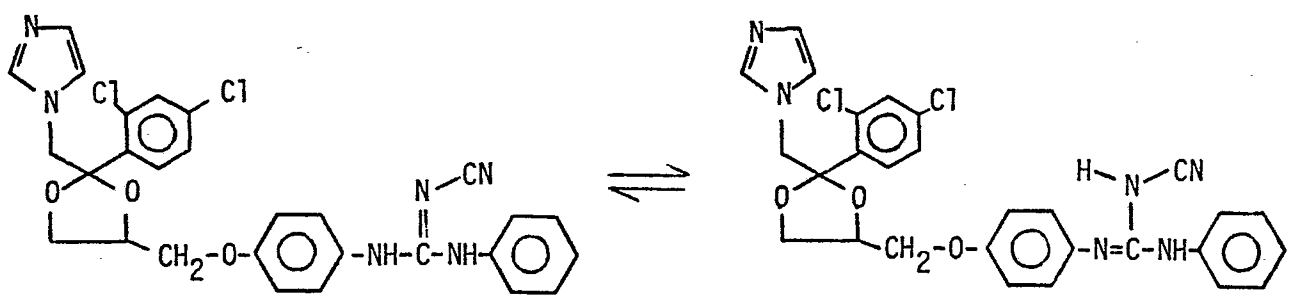

Die Herstellung erfolgt analog Beispiel 16 mit Anilin.

Farblose Kristalle vom Schmelzpunkt 78 - 79 °C

Rf = 0.88 (Essigester / $CH_3OH$ 80 : 20)

Ausbeute: 2.93 g (52 % d.Th.)

$C_{28}H_{24}Cl_2N_2O_3$ (563)

| | | | |
|---|---|---|---|
| Ber.: | C 59.69 | H 4.29 | N 14.91 |
| Gef.: | C 59.02 | H 4.76 | N 14.25 |

IR ($cm^{-1}$): 2185 ($\nu$-CN)

$^1$H-NMR-Spektrum: (CDCl$_3$, TMS als interner Standard) $\delta$ = 3.47 - 4.00 (m) (2xC$\underline{H}_2$) 4 H,
4.27 (m) (C$\underline{H}$) 1 H,
4.43 (s) (C$\underline{H}_2$) 2 H,
6.70 - 7.93 (m) (Aromaten-$\underline{H}$, N$\underline{H}$) 17 H ppm.

## Beispiel 27

cis-N'-[4-[2-(2,4-Dichlorphenyl)-2-(1H-imidazol-1-ylmethyl)-1,3-dioxolan-4-ylmethoxy]-phenyl]-N''-Cyano-N'''-benzylguanidin

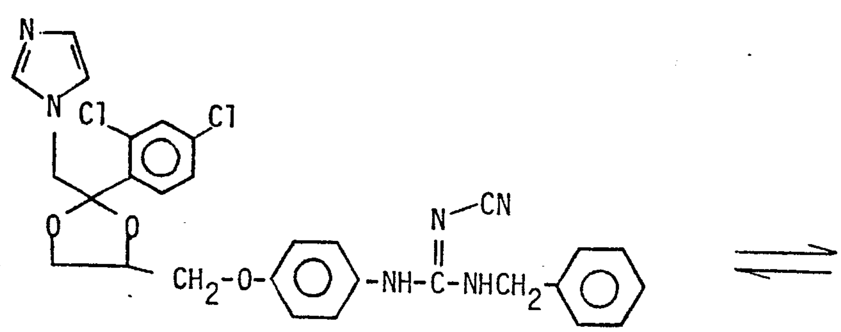

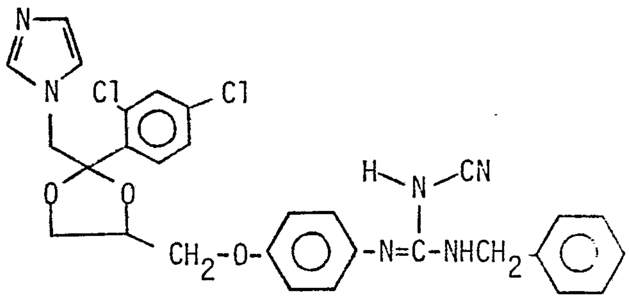

Die Herstellung erfolgt analog Beispiel 16 mit Benzylamin.

Farblose Kristalle vom Schmelzpunkt 75 - 76 °C

Rf = 0.72 (Essigester / $CH_3OH$ 90 : 10)

Ausbeute: 2.60 g (45 % d.Th.)

$C_{29}H_{26}Cl_2N_6O_3$ (577)

IR ($cm^{-1}$): 2180 ($\nu$-CN)

$^1H$-NMR-Spektrum: (d$_6$-DMSO, TMS als interner Standard)

$\delta$ = 3.53 - 4.00 (m) (2x$CH_2$) 4 H,
4.40 (m) ($CH_2$, CH) 3 H,
4.53 (s) ($CH_2$) 2 H,
6.67 - 7.73 (m) (Aromaten-H, N-H) 16 H,
9.00 (s) (N-H) (austauschbar mit $D_2O$) 1 H ppm.

Beispiel 28

cis-N'[4-[2-(2,4-Dichlorphenyl)-2-(1H-imidazol-1-ylmethyl)-1,3-dioxolan-4-ylmethoxy]-phenyl]-N''-cyano-N'''-4-chlorbenzylguanidin

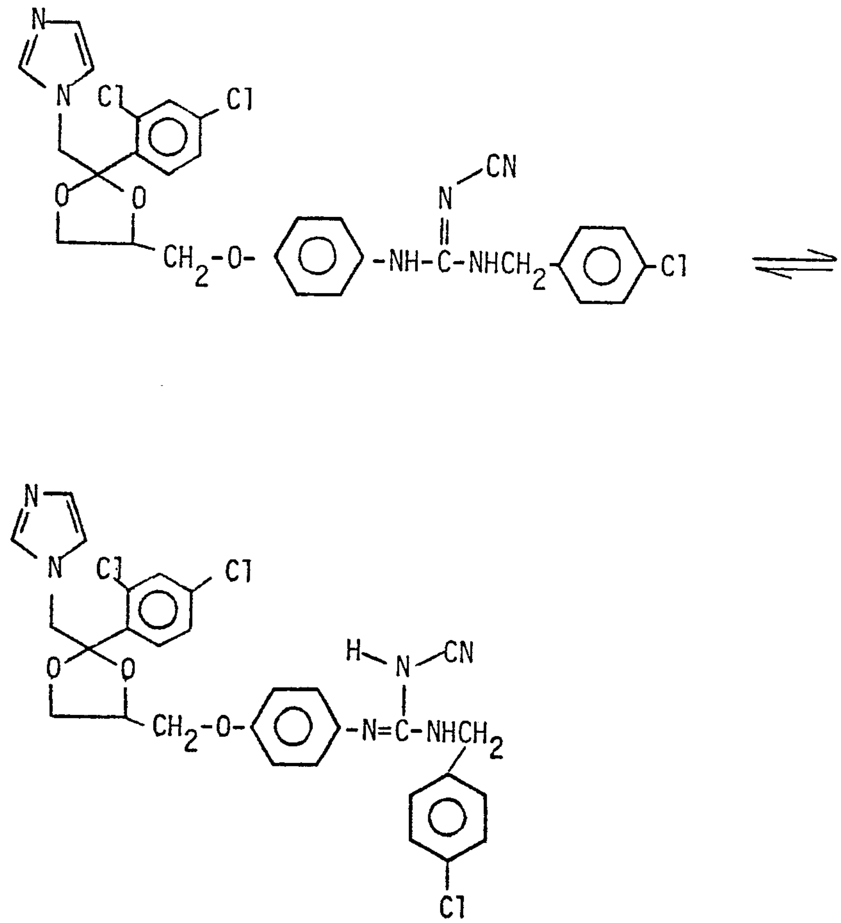

Die Herstellung erfolgt analog Beispiel 16 mit 4-Chlorbenzylamin.

Farblose Kristalle vom Schmelzpunkt 95 °C

Rf = 0.80 (Essigester / $CH_3OH$ 80 : 20)

Ausbeute: 3.42 g (56 % d.Th.)

| | | | | |
|---|---|---|---|---|
| $C_{29}H_{25}Cl_3N_6O_3$ (612) | Ber.: | C 56.92 | H 4.12 | N 13.73 |
| | Gef.: | C 56.72 | H 4.45 | N 13.51 |

IR ($cm^{-1}$): 2180 ($\nu$-CN)

$^1$H-NMR-Spektrum: ($d_6$-DMSO, TMS als interner Standard)

$\delta$ = 3.57 (d) ($C\underline{H}_2$) 2 H,
3.67 - 4.00 (m) ($C\underline{H}_2$) 2 H,
4.37 (m) (2x$C\underline{H}_2$, C$\underline{H}$) 5 H,
6.57 (t) (N-$\underline{H}$) (austauschbar mit $D_2O$) 1 H,
6.67 - 7.60 (m) (Aromaten-$\underline{H}$) 14 H,
7.70 (s) (N-$\underline{H}$) (austauschbar mit $D_2O$) 1 H ppm.

Beispiel 29

cis-N'-[4-[2-(2,4-Dichlorphenyl)-2-(1H-imidazol-1-ylmethyl)-1,3-dioxolan-4-ylmethoxy]-phenyl]-N''-cyano-N'''-dimethylguanidin

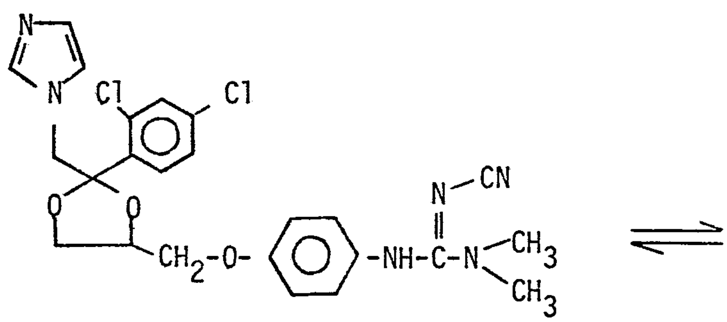

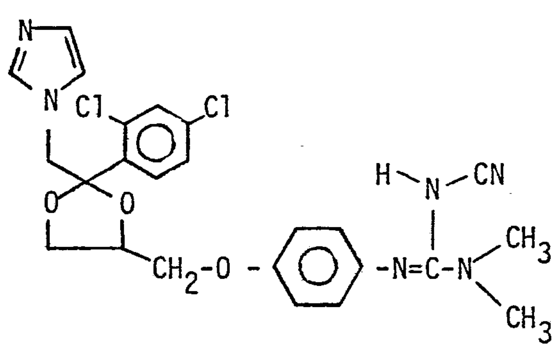

Die Herstellung erfolgt analog Beispiel 16 mit Dimethylamin.

Farblose Kristalle vom Schmelzpunkt 98 - 99 °C

Rf = 0.70 (Dichlorethan / $CH_3OH$ 80 : 20)

Ausbeute: 4.53 g ( 88 % d.Th.)

$C_{24}H_{24}Cl_2N_6O_3$ (515)

IR ($cm^{-1}$) : 2180 ($\nu$-CN)

$^1$H-NMR-Spektrum: (CDCl$_3$, TMS als interner Standard)

$\delta$ = 2.90 (s) ($N(CH_3)_2$) 6 H,
3.23 (dd) (C$\underline{H}$) 1 H,
3.50 - 4.00 (m) (C$\underline{H}_2$, C$\underline{H}$) 3 H,
4.30 (m) (C$\underline{H}$) 1 H,
4.43 (s) (C$\underline{H}_2$) 2 H,
6.67 - 7.70 (m) (Aromaten-$\underline{H}$,N-$\underline{H}$) 11 H ppm.

## Beispiel 30

cis-N'-[4-[2-(2,4-Dichlorphenyl)-2-(1H-imidazol-1-ylmethyl)-1,3-dioxolan - 4-ylmethoxy]-phenyl]-N''-cyano-N'''-diethylguanidin

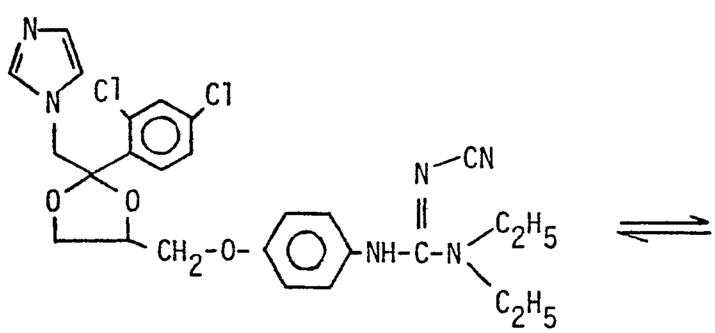

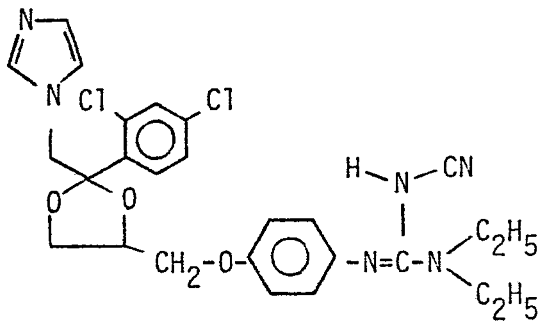

Die Herstellung erfolgt analog Beispiel 16 mit Diethylamin.

Farblose Kristalle vom Schmelzpunkt 85 - 86 °C

Rf = 0.23 (Essigester / $CH_3OH$ 80 : 20)

Ausbeute: 2.00 g (37 % d.Th.)

$C_{26}H_{28}Cl_2N_6O_3$ (543)

IR ($cm^{-1}$) : 2180 ($\nu$-CN)

$^1$H-NMR-Spektrum: (CDCl$_3$, TMS als interner Standard)

$\delta$ = 1.13 (t) (2x$CH_3$) 6 H,
3.33 (m) (2x$CH_2$, CH) 5 H,
3.53 - 4.00 (m) ($CH_2$, CH) 3 H,
4.33 (m) (CH) 1 H,
4.43 (s) ($CH_2$) 2 H,
6.60 - 7.67 (m) (Aromaten-H) 10 H,
7.93 (breit) (N-H) (austauschbar mit $D_2O$) 1 H ppm.

Beispiel 31

cis-N'-[4-[2-(2,4-Dichlorphenyl)-2-(1H-imidazol-1-ylmethyl)-1,3-dioxolan-4-ylmethoxy]-phenyl]-N''-cyano-N'''-tetramethylenguanidin

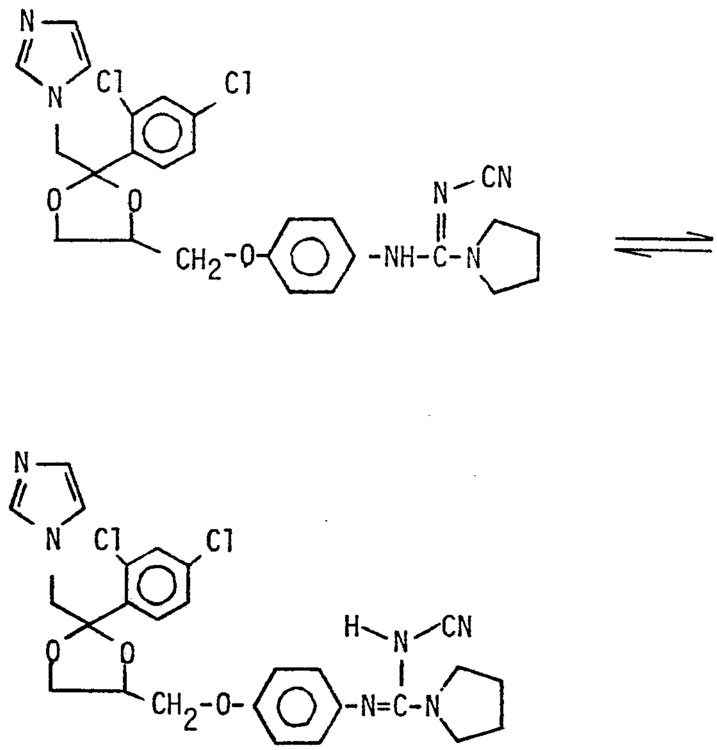

Die Herstellung erfolgt analog Beispiel 16 mit Pyrrolidin.

Farblose Kristalle vom Schmelzpunkt 97 - 98 °C

Rf = 0.46 (Dichlorethan / $CH_3OH$ 80 : 20)

Ausbeute: 4.17 g (77 % d.Th.)

| | | | | |
|---|---|---|---|---|
| $C_{26}H_{26}Cl_2N_6O_3$ (541) | Ber.: | C 57.68 | H 4.84 | N 15.52 |
| | Gef.: | C 57.83 | H 4.85 | N 15.03 |

IR ($cm^{-1}$): 2175 ($\nu$-CN)

$^{1}$H-NMR-Spektrum: ($CDCl_3$, TMS als interner Standard)

$\delta$ = 1.87 (m) (2x$CH_2$) 4 H,
3.40 (m) (2x$CH_2$, CH) 5 H,
3.53 - 4.00 (m) ($CH_2$, CH) 3 H
4.33 (m) (CH) 1 H,
4.43 (s) ($CH_2$) 2 H,
6.67 - 7.70 (m) (Aromaten-H) 10 H,
7.80 (s) (N-H) (austauschbar mit $D_2O$) 1 H ppm.

## Beispiel 32

cis-N'-[4-[2-(2,4-Dichlorphenyl)-2-(1H-imidazol-1-ylmethyl)-1,3-dioxolan-4-ylmethoxy]-phenyl]-N''-cyano-N'''-tetramethylenoxyguanidin

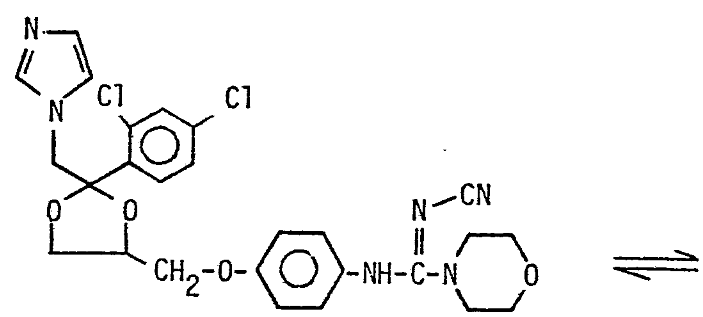

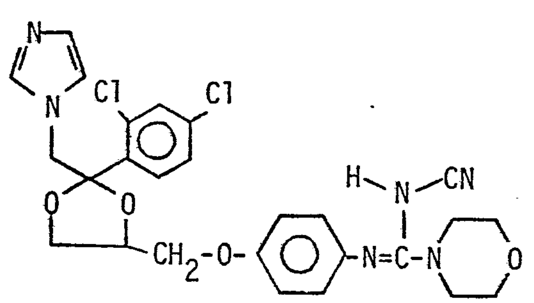

Die Herstellung erfolgt analog Beispiel 16 mit Morpholin.

Farblose Kristalle vom Schmelzpunkt 89 - 90 °C

Rf = 0.30 (Essigester / $CH_3OH$ 80 : 20)

Ausbeute: 4.07 g (73 % d.Th.)

$C_{26}H_{26}Cl_2N_6O_4$ (557)

IR ($cm^{-1}$): 2180 ($\nu$-CN)

$^1$H-NMR-Spektrum: (d$_6$-DMSO, TMS als interner Standard)

$\delta$ = 3.33 - 4.00 (m) 12 H,
4.40 (m) (C<u>H</u>) 1 H,
4.57 (s) (C<u>H</u>$_2$) 2 H,
6.67 - 7.77 (m) (Aromaten-<u>H</u>) 10 H,
9.20 (breit) (austauschbar mit $D_2O$) 1 H ppm.

## Beispiel 33

cis-N'-[4-[2-(2,4-Dichlorphenyl)-2-(1H-imidazol-1-ylmethyl)-1,3-dioxolan-4-ylmethoxy]-phenyl]-N''-cyano-N'''-tetramethylen-4-ylmethyl-guanidin

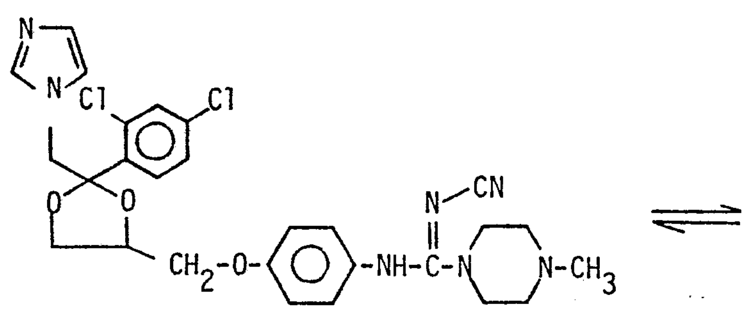

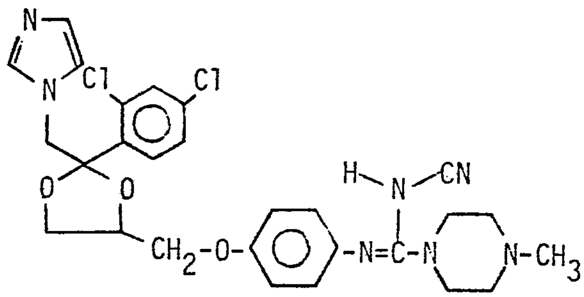

Die Herstellung erfolgt analog Beispiel 16 mit 1-Methylpiperazin.

Farblose Kristalle vom Schmelzpunkt 170 - 171 $^{o}$ C.

Rf = 0.59 (Dichlorethan / $CH_3OH$ 80 : 20)

Ausbeute: 4.45 g (78 % d.Th.)

$C_{27}H_{29}Cl_2N_7O_3$ (570)

IR ($cm^{-1}$): 2180 ($\nu$-CN)

$^1$H-NMR-Spektrum: (CDCl$_3$, TMS als interner Standard)

$\delta$ = 2.30 (s) ($CH_3$) 3 H,
2.40 (m) ($2xCH_2$) 4 H,
3.40 - 4.07 (m) 8 H,
4.40 (m) ($CH_2$, CH) 3 H,
6.67 - 7.73 (m) (Aromaten-H, N-H) 11 H ppm.

## Beispiel 34

cis-N'-[4-[2-(2,4-Dichlorphenyl)-2-(1H-imidazol-1-ylmethyl)-1,3-dioxolan-4-ylmethoxy]-phenyl]-N''-cyano-N'''-tetramethylen-4-ylacetyl-guanidin

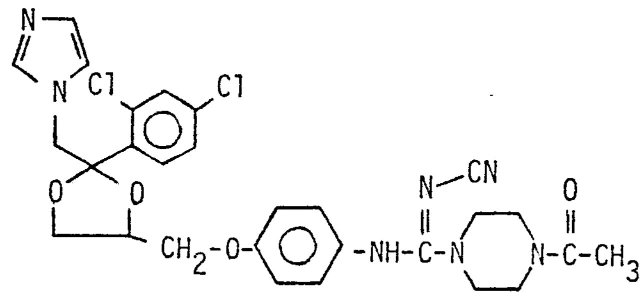

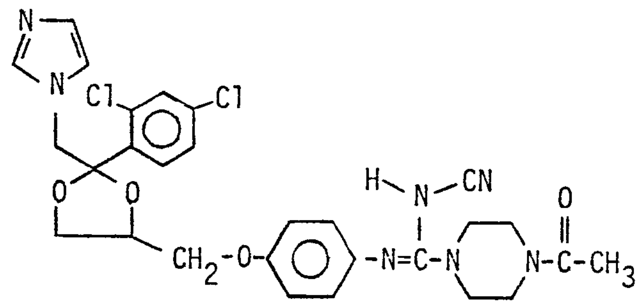

Die Herstellung erfolgt analog Beispiel 16 mit 1-Acetylpiperazin.

Farblose Kristalle vom Schmelzpunkt 117 - 118 °C

Rf = 0.38 (Dichlorethan / $CH_3OH$ 80 : 20)

Ausbeute: 2.81 g (47 % d.Th.)

$C_{28}H_{29}Cl_2N_7O_4$ (599) Ber.: C 56.19 H 4.88 N 16.38

Gef.: C 55.66 H 4.78 N 16.04

IR ($cm^{-1}$): 2175 ($\nu$-CN)

$^1$H-NMR-Spektrum: (CDCl$_3$, TMS als interner Standard) $\delta$ = 2.07 (s) ($CH_3$) 3 H,
3.23 - 3.97 (m) 12 H,
4.37 (m) (CH) 1 H,
4.47 (s) ($CH_2$) 2 H,
6.63 - 7.70 (m) (Aromaten-H) 10 H,
8.63 (s) (N-H) (austauschbar mit $D_2O$) 1 H ppm.

Beispiel 35

cis-N'-[3-[2-(2,4-Dichlorphenyl)-2-(1H-imidazol-1-ylmethyl)-1,3-dioxolan 4-ylmethoxy]-phenyl]-N''-cyano-N'''-tetramethylen-4-ylacetyl-guanidin

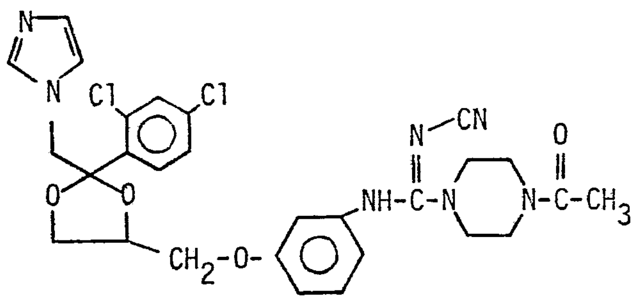

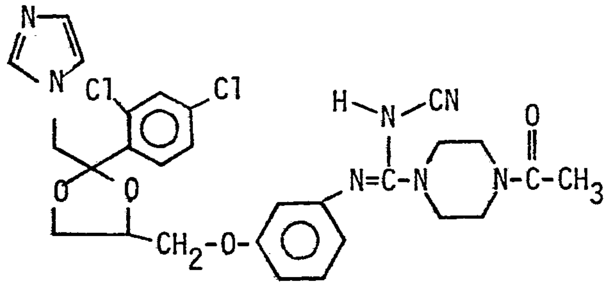

Die Herstellung erfolgt analog Beispiel 16 mit Acetylpiperazin.

Farblose Kristalle vom Schmelzpunkt 101 - 102 °C

Rf = 0.76 ($CHCl_3$ / $CH_3OH$ / $NH_3$ 95:15:2)

Ausbeute: 2.57 g (43 % d.Th.)

$C_{28}H_{29}Cl_2N_7O_4$ (599)

IR ($cm^{-1}$): 2185 ($\gamma$-CN)

$^1$H-NMR-Spektrum: (d$_6$-DMSO, TMS als interner Standard)

$\delta$ = 2.00 (s) ($CH_3$) 3 H,
3.23 - 4.00 (m) 12 H,
4.33 (m) (CH) 1H,
4.53 (s) ($CH_2$) 2 H,
6.50 - 7.73 (m) (Aromaten-H) 10 H,
9.33 (breit) (N-H) (austauschbar mit $D_2O$) 1 H ppm.

Beispiel 36

5-[4-[2-(2,4-Dichlorphenyl)-2-(1H-imidazol-1-ylmethyl)-1,3-dioxolan-4-ylmethoxy]-1-aminophenyl]-3-amino-1-H-1,2,4-triazol

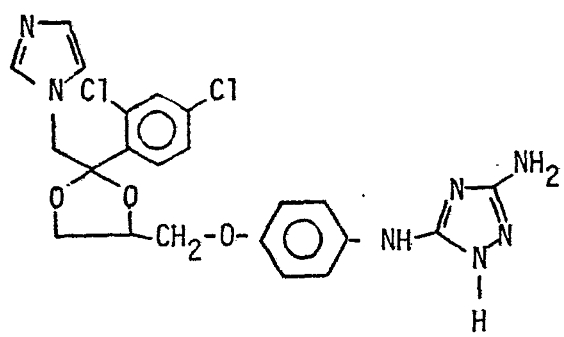

2.0 g (3.5 mmol) cis-N'-[4-[2-(2,4-Dichlorphenyl)-2-(1H-imidazol-1-ylmethyl)-1,3-dioxolan-4-ylmethoxy]-phenyl]-N''-cyano-phenylisoharnstoff (Beispiel 1) werden in 50 ml Ethanol gelöst und mit 0.35 g (7 mmol) Hydrazinhydrat (80 %) 1 h auf Rückflußtemperatur erhitzt. Nach dem Abkühlen der Reaktionslösung wird der Feststoff aus Isopropanol umkristallisiert.

Farblose Kristalle vom Schmelzpunkt 210 - 211 °C

Rf = 0.12 (Essigester / $CH_3OH$ 80 : 20)

Ausbeute: 1.37 g (78 % d.Th.)

| | | | | |
|---|---|---|---|---|
| $C_{22}H_{21}Cl_2N_7O_3$ (502) | Ber.: | C 52.60 | H 4.21 | N 19.52 |
| | Gef.: | C 52.53 | H 4.44 | N 19.27 |

$^1$H-NMR-Spektrum: (d$_6$-DMSO, TMS als interner Standard)

$\delta$ = 3.33 - 4.00 (m) (2xC$\underline{H}_2$) 4 H,
4.33 (m) (C$\underline{H}$) 1 H,
4.53 (s) (C$\underline{H}_2$) 2 H,
5.73 (s) (N$\underline{H}_2$) (austauschbar mit $D_2O$) 2 H,
6.60 - 7.73 (m) (Aromaten-$\underline{H}$) 10 H,
8.37 (s) (N-$\underline{H}$) (austauschbar mit $D_2O$) 1 H,
11.10 (s) (N-$\underline{H}$) (austauschbar mit $D_2O$) 1 H ppm.

Beispiel 37

5-[3-[2-(2,4-Dichlorphenyl)-2-(1H-imidazol-1-ylmethyl)-1,3-dioxolan-4-ylmethoxy]-1-aminophenyl]-3-amino-1-H-1,2,4-triazol

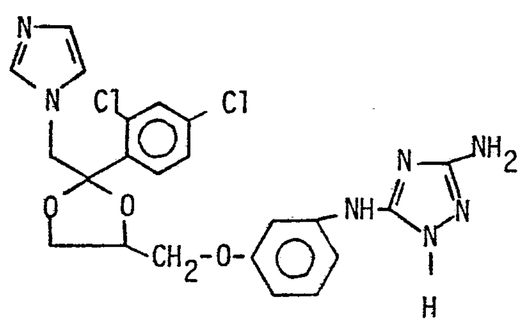

Die Herstellung erfolgt analog Beispiel 36 mit Hydrazinhydrat.

Farblose Kristalle vom Schmelzpunkt 123 - 124 °C

Rf = 0.10 (Essigester / $CH_3OH$ 80 : 20)

Ausbeute: 3.77 g (75 % d.Th.)

$C_{22}H_{21}Cl_2N_7O_3$ (502)

$^1$H-NMR-Spektrum: (d$_6$-DMSO, TMS als interner Standard)

$\delta$ = 3.47 - 4.00 (m) (2x$CH_2$) 4H,
4.37 (m) (C<u>H</u>) 1 H,
4.53 (s) (C<u>H</u>$_2$) 2 H,
5.77 (breit) (N<u>H</u>$_2$) (austauschbar mit $D_2O$) 2 H,
6.27 (m) (Aromaten-<u>H</u>) 1 H,
6.80 - 7.73 (m) (Aromaten-<u>H</u>) 9 H,
8.63 (s) (N-<u>H</u>) (austauschbar mit $D_2O$) 1 H,
11.10 (s) (N-<u>H</u>) (austauschbar mit $D_2O$) 1 H ppm.

Beispiel 38

cis-5-[4-[2-(2,4-Dichlorphenyl)-2-(1H-imidazol-1-ylmethyl)-1,3-dioxolan-4-ylmethoxy]-1-aminophenyl]-3-amino-1-methyl-1H-1,2,4-triazol

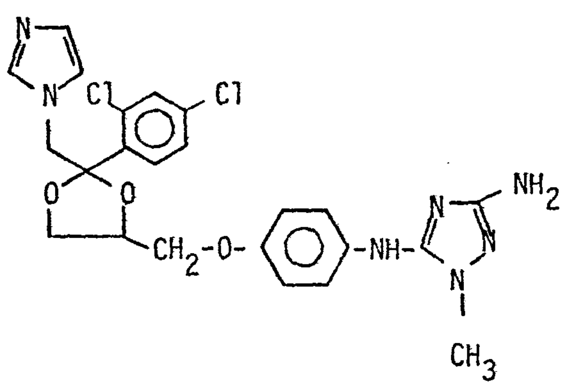

Die Herstellung erfolgt analog Beispiel 36 mit Methylhydrazin.

Farblose Kristalle vom Schmelzpunkt 100 - 101 °C

Rf = 0.50 ($CHCl_3$ / $CH_3OH$ / $NH_3$ 90 : 15 : 2 )

Ausbeute: 3.20 g (62 % d.Th.)

$C_{23}H_{23}Cl_2N_7O_3$ (516) Ber.: C 53.49 H 4.49 N 18.99

Gef.: C 53.90 H 4.58 N 18.62

$^1$H-NMR-Spektrum: (d$_6$-DMSO, TMS als interner Standard) $\delta$ = 3.17 - 4.00 (m) (2x$CH_2$) 4 H,
3.47 (s) (N-$CH_3$) 3 H,
4.33 (m) (CH) 1 H,
4.53 (s) ($CH_2$) 2 H,
4.93 (s, breit) $NH_2$)(austauschbar mit $D_2O$) 2 H,
6.63 - 7.80 (m) (Aromaten-H) 10 H,
8.43 (s) (N-H) (austauschbar mit $D_2O$) 1 H ppm.

a) Herstellung von 1-Methyl-N$^5$-(4-hydroxyphenyl)-1H-1,2,4-triazol-3,5-diamin

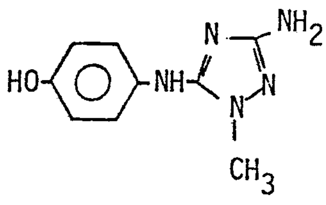

10.3 g (0.05 mol) N''-Cyano-N'-(4-hydroxyphenyl)-methylisothioharnstoff (s. Beispiel 5a) und 4.6 g (0.1 mol) Methylhydrazin werden in 50 ml Isopropanol gelöst und 1 Stunde auf Rückflußtemperatur erhitzt. Der angefallene Feststoff wird aus Methanol kristallisiert.

Farblose Kristalle vom Schmelzpunkt 270 - 271 °C

b) Herstellung von cis-5-[4-[2-(2,4-Dichlorphenyl)-2-(1H-imidazol-1-yl-methyl)-1,3-dioxolan-4-ylmethoxy]-1-aminophenyl]-3-amino-1-methyl-1H-1,2,4-triazol

Die Herstellung erfolgt analog Beispiel 5 b.

Die physikalischen Daten der erhaltenen Verbindung stimmen mit denen von Beispiel 38 überein.

Beispiel 39

cis-5-[3-[2-(2,4-Dichlorphenyl)-2-(1H-imidazol-1-ylmethyl)-1,3-dioxolan-4-ylmethoxy]-1-aminophenyl]-3-amino-1-methyl-1H-1,2,4-triazol

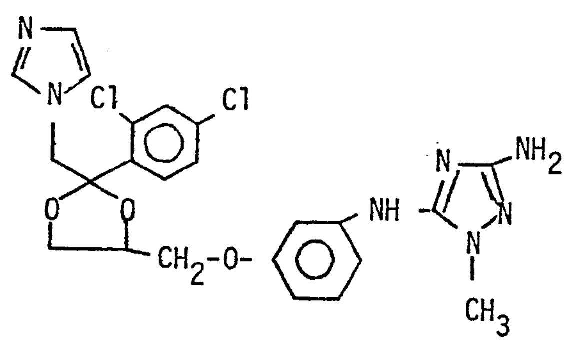

Die Herstellung erfolgt analog Beispiel 36 mit Methylhydrazin.

Farblose Kristalle vom Schmelzpunkt 95 - 97 °C

Rf = 0.52 (Diisopropylether / $CH_3OH$ 90 : 20)

Ausbeute: 3.46 g (67 % d.Th.)

$C_{23}H_{23}Cl_2N_7O_3$ (516) Ber.: C 53.49 H 4.49 N 18.99

Gef.: C 53.02 H 4.53 N 19.15

$^1$H-NMR-Spektrum: ($CDCl_3$, TMS als interner Standard)

$\delta$ = 3.10 - 4.00 (m) (2x$C\underline{H}_2$) 4 H,
3.53 (s) (N-$C\underline{H}_3$) 3 H,
4.33 (m) (C$\underline{H}$) 1 H,
4.43 (s) ($C\underline{H}_2$) 2 H,
6.27 - 7.67 (m) (Aromaten-$\underline{H}$, $N\underline{H}_2$) 12 H,
7.90 (s) (N-$\underline{H}$) (austauschbar mit $D_2O$) 1 H ppm.

## Beispiel 40

cis-5-[4-[2-(2,4-Dichlorphenyl)-2-(1H-1,2,4-triazol-1-ylmethyl)-1,3-dioxolan-4-ylmethoxy]-1-aminophenyl]-3-amino-1-methyl-1H-1,2,4-triazol

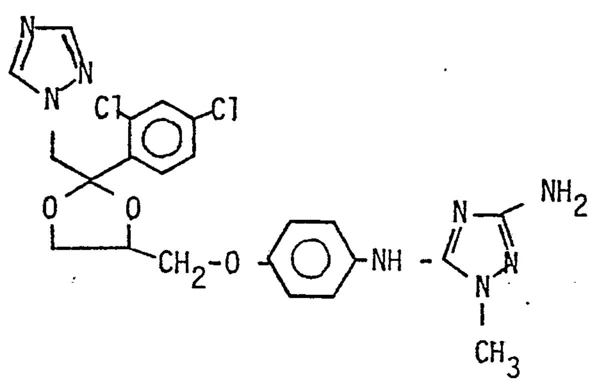

Die Herstellung erfolgt analog Beispiel 36 mit Methylhydrazin.

Farblose Kristalle vom Schmelzpunkt 103 - 104 °C

Rf = 0.10 ($CHCl_3$ / EtOH 90 : 10)

Ausbeute: 3.62 g (70 % d.Th.)

$C_{22}H_{22}Cl_2N_8O_3$ (517)

$^1$H-NMR-Spektrum: (d$_6$-DMSO, TMS als interner Standard)

δ = 3.47 (s) (N-CH$_3$) 3 H,
3.60 - 4.00 (m) (2xCH$_2$) 4 H,
4.33 (m) (CH) 1 H,
4.80 (s) (CH$_2$) 2 H,
4.93 (s) (NH$_2$) (austauschbar mit D$_2$O) 2 H,
6.70 - 7.73 (m) (Aromaten-H) 7 H,
7.87 (s) (Aromaten-H) 1 H,
8.47 (s) (Aromaten-H, N-H) 2 H ppm.

Beispiel 41

cis-5-[3-[2-(2,4-Dichlorphenyl)-2-(1H-1,2,4-triazol-1-ylmethyl)-1,3-dioxolan-4-ylmethoxy]-1-aminophenyl]-3-amino-1-methyl-1H-1,2,4-triazol

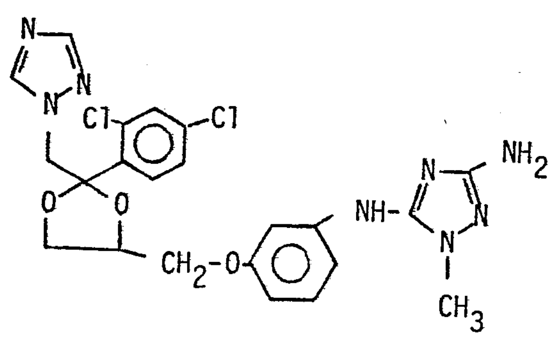

Die Herstellung erfolgt analog Beispiel 36 mit Methylhydrazin.

Farblose Kristalle vom Schmelzpunkt 180 - 182 °C.

Rf = 0.35 ($CHCl_3$ / EtOH 90 : 10)

Ausbeute: 3.52 g (68 % d.Th.)

$C_{22}H_{22}Cl_2N_8O_3$ (517)

$^1$H-NMR-Spektrum: (d$_6$-DMSO, TMS als interner Standard)

$\delta$ = 3.13 - 4.07 (m) (2x$CH_2$) 4 H,
3.50 (s) (N-$CH_3$) 3 H,
4.40 (m) (CH) 1 H,
4.83 (s) ($CH_2$) 2 H,
5.03 (breit) ($NH_2$) (austauschbar mit $D_2O$) 2 H,
6.33 - 7.70 (m) (Aromaten-H) 7 H,
7.83 (s) (Aromaten-H) 1 H,
8.40 (s) (Aromaten-H) 1 H,
8.67 (s) (N-H), (austauschbar mit $D_2O$) 1 H ppm.

## Beispiel 42

cis-3-[4-[2-(2,4-Dichlorphenyl)-2-(1H-imidazol-1-ylmethyl)-1,3-dioxolan-4-ylmethoxy]-1-aminophenyl]-5-amino-1,2,4-oxadiazol

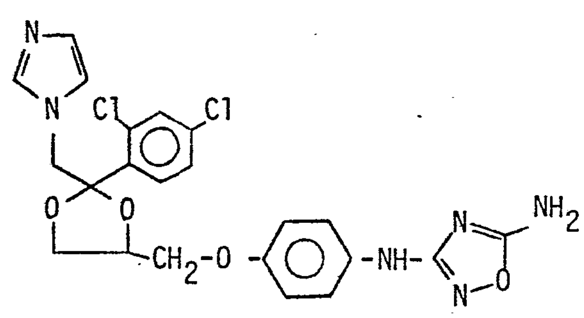

Die Herstellung erfolgt analog Beispiel 36 mit Hydroxylaminhydrochlorid und Triethylamin.

Farblose Kristalle vom Schmelzpunkt 82 - 83 °C

Rf = 0.33 (Essigester/Methanol 80 : 20)

Ausbeute: 1.86 g (37 % d.Th.)

$C_{22}H_{20}N_6O_4Cl_2$ (503)

$^1$H-NMR-Spektrum: (d$_6$-DMSO, TMS als interner Standard)

$\delta$ = 3.37 - 4.00 (m) (2x$CH_2$) 4 H,
4.33 (m) (CH) 1 H,
4.50 (s) ($CH_2$) 2 H,
6.67 - 7.70 (m) (Aromaten-H) 10 H,
7.50 (s) ($NH_2$) (austauschbar mit $D_2O$) 2 H,
9.00 (s) (N-H) (austauschbar mit $D_2O$) 1 H ppm.

Beispiel 43

cis-4,6-Diamino-2-[4-[[2-(2,4-dichlorphenyl)-2-(1H-imidazol-1-ylmethyl)-1,3-dioxolan-4-ylmethoxy]-1-aminophenyl]-1,3,5-triazin

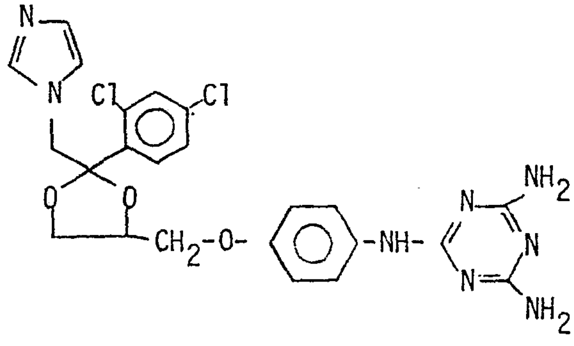

Die Herstellung erfolgt analog Beispiel 36 mit Guanidin-Hydrochlorid und Natriumethanolat.

Farblose Kristalle vom Schmelzpunkt 121 - 122 °C

Rf = 0.43 (Chloroform / $CH_3OH$ / $NH_3$ 23:3:3)

Ausbeute: 2.01 g (38 % d.Th.)

$C_{23}H_{22}N_8O_3Cl_2$ (529)

$^1$H-NMR-Spektrum: (d$_6$-DMSO, TMS als interner Standard)

$\delta$ = 3.37 - 4.00 (m) (2xC$\underline{H}_2$) 4 H,
4.33 (m) (-C$\underline{H}$) 1 H,
4.50 (s) (C$\underline{H}_2$) 2 H,
6.23 (s) (2xN$\underline{H}_2$) (austauschbar mit $D_2O$) 4 H,
6.63 - 7.77 (m) (Aromaten-$\underline{H}$) 10 H,
8.67 (s) (N-$\underline{H}$) (austauschbar mit $D_2O$) 1 H ppm.

Beispiel 44

cis-1-Cyanimino-methylthio-methylen-4-[4[[2-(2,4-dichlorphenyl)-2-(1H-imidazol-1-ylmethyl)-1,3-dioxolan-4-yl]methoxy]-phenyl]-piperazin

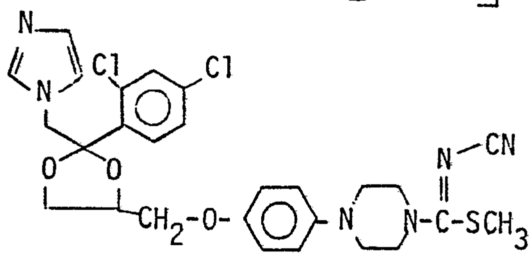

Die Herstellung erfolgt analog Beispiel 5 b aus 4-(4-Hydroxyphenyl)-1-(N-cyanimino-methylthiomethylen)-piperazin und cis-2-(2,4-Dichlorphenyl)-2-(1H-imidazol-1-ylmethyl)-1,3-dioxolan-4-ylmethyl-methansulfonat.

Farblose Kristalle vom Schmelzpunkt 142 - 144 °C

Rf = 0.63 (Essigester / $CH_3OH$ 80 : 20)

| | | | | |
|---|---|---|---|---|
| $C_{27}H_{28}Cl_2N_6O_3S$ (588) | Ber.: | C 55,20 | H 4,80 | N 14,30 |
| | Gef.: | C 55,26 | H 4,87 | N 14,16 |

IR ($cm^{-1}$): 2190 ($\nu$-CN)

$^1$H-NMR-Spektrum: (d$_6$-DMSO, TMS als interner Standard)

$\delta$ = 2.73 (s) (S-CH$_3$) 3 H,
3.13 (m) (2xCH$_2$) 4 H,
3.63 (m) (2xCH$_2$) 4 H,
3.90 (m) (2xCH$_2$) 4 H,
4.33 (m) (CH) 1 H,
4.50 (s) (CH$_2$) 2 H,
6.70 - 7.70 (m) (Aromaten-H) 10 H ppm.

Beispiel 45

cis-Cyanimino-phenoxy-methylen-4-[4[[2-(2,4-dichlorphenyl)-2-(1H-imidazol-1-ylmethyl)-1,3-dioxolan-4-yl]methoxy]-phenyl]-piperazin-Hydrochlorid

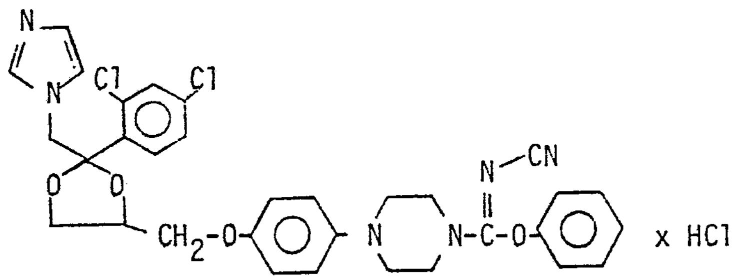

Die Herstellung erfolgt analog Beispiel 5 b aus 4-(4-Hydroxyphenyl)-1-(N-cyanimino-phenoxy-methylen)-piperazin und cis-2-(2,4-Dichlorphenyl)-2-(1H-imidazol-1-ylmethyl)-1,3-dioxolan-4-ylmethyl-methansulfonat.

Farblose Kristalle vom Schmelzpunkt 154 - 155 °C

Rf = 0.47 (Diisopropylether / $CH_3OH$ 70 : 30)

$C_{32}H_{31}Cl_3N_6O_4$ (669)

IR ($cm^{-1}$): 2200 ($\nu$-CN)

$^1$H-NMR-Spektrum: (d$_6$-DMSO, TMS als interner Standard)

$\delta$ = 3.57 (m) (2x$CH_2$) 4 H,
3.87 (m) (2x$CH_2$) 4 H,
4.07 (m) (2x$CH_2$) 4 H,
4.40 (m) (CH) 1 H,
4.87 (s) ($CH_2$) 2 H,
6.87 - 7.80 (m) (Aromaten-H, -H) 15 H,
9.20 (s) (Aromaten-H) 1 H ppm.

Beispiel 46

cis-1-[(1-Methyl-3-amino)-1,2,4-triazol-5-yl]-4-[4[[2-(2,4-dichlorphenyl)-2-(1H-imidazol-1-ylmethyl)-1,3-dioxolan-4-yl]-methoxy]phenyl]piperazin

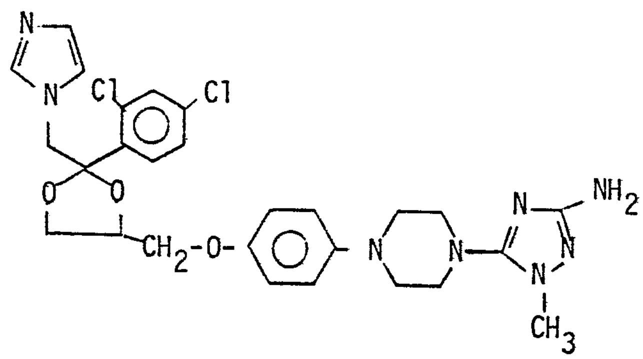

Die Herstellung erfolgt analog Beispiel 5 b aus 4-(4-Hydroxyphenyl)-1-(1-methyl-3-amino-1,2,4-triazol-5-yl)-piperazin und cis-2-(2,4-Dichlorphenyl)-2-(1H-imidazol-1-ylmethyl)-1,3-dioxolan-4-ylmethyl-methansulfonat.

Farblose Kristalle vom Schmelzpunkt 194 °C

Rf = 0.19 (Essigester / $CH_3OH$ 80 : 20 )

$C_{27}H_{30}Cl_2N_8O_3$ (586)

$^1$H-NMR-Spektrum: (d$_6$-DMSO, TMS als interner Standard)

$\delta$ = 3.13 (m) (2x$CH_2$) 4 H,
3.43 (s) ($CH_3$) 3 H,
3.63 (m) (2x$CH_2$) 4 H,
3.87 (m) (2x$CH_2$) 4 H,
4.33 (m) (CH) 1 H,
4.53 (s) ($CH_2$) 2 H,
5.00 (breit) ($NH_2$) 2 H, austauschbar mit $D_2O$
6.67 - 7.73 (m) (Aromaten-H) 10 H ppm.

Beispiel 47

cis-1-Cyanimino-methylthio-methylen-4-[4[[2-(2,4-dichlorphenyl)-2-(1H-1,2,4-triazol-1-ylmethyl)-1,3-dioxolan-4-yl]methoxy]-phenyl]-piperazin

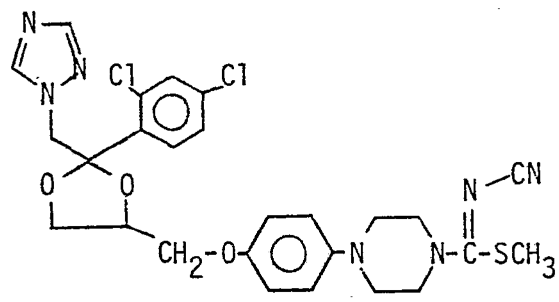

Die Herstellung erfolgt analog Beispiel 5 b aus 4-(4-Hydroxyphenyl)-1-(N-cyanimino-methylthiomethylen)-piperazin und cis-2-(2,4-Dichlorphenyl)-2-(1H-1,2,4-triazol-1-ylmethyl)-1,3-dioxolan-4-yl-methyl-methansulfonat.

Farblose Kristalle vom Schmelzpunkt 67 - 68 $^{o}$ C

Rf = 0.63 (Essigester / $CH_3OH$ 90 : 10)

| | | | | |
|---|---|---|---|---|
| $C_{26}H_{27}Cl_2N_7O_3S$ (588) | Ber.: | C 53.06 | H 4.62 | N 16.66 |
| | Gef.: | C 53.43 | H 4.67 | N 16.66 |

IR ($cm^{-1}$): 2190 ($\nu$-CN)

$^1$H-NMR-Spektrum: (CDCl$_3$, TMS als interner Standard)

$\delta$ = 2.77 (s) (S-$CH_3$) 3 H,
3.12 (m) (2 x $CH_2$) 4 H,
3.57 - 4.17 (m) (2 x $CH_2$, 2 x $OCH_2$) 8 H,
4.37 (m) (CH) 1 H,
4.82 (s) ($CH_2$) 2 H,
6.70 - 7.70 (m) (Aromaten-H) 7 H,
7.90 (s) (Aromaten-H) 1 H,
8.27 (s) (Aromaten-H) 1 H ppm.

Beispiel 48

cis-1-Cyanimino-phenoxy-methylen-4-[4[[2-(2,4-dichlorphenyl)-2-(1H-1,2,4-triazol-1-ylmethyl)-1,3-dioxolan-4-yl] methoxy]-phenyl]-piperazin

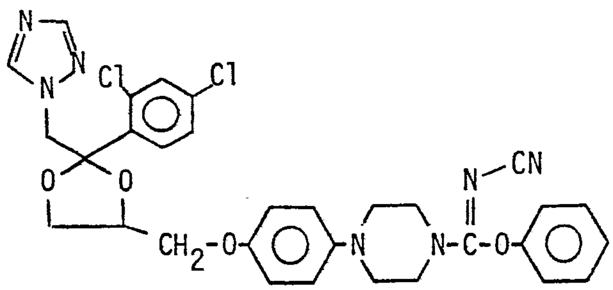

Die Herstellung erfolgt analog Beispiel 5 b aus 4-(4-Hydroxyphenyl)-1-(N-cyanimino-phenoxymethylen)-piperazin und cis-2-(2,4-Dichlorphenyl)-2-(1H-1,2,4-triazol-1-ylmethyl)-1,3-dioxolan-4-ylmethyl-methansulfonat.

Farblose Kristalle vom Schmelzpunkt 129 °C

Rf = 0.82 (Essigester/$CH_3OH$ 80 : 20)

$C_{31}H_{29}Cl_2N_7O_4$ (635)

IR ($cm^{-1}$): 2200 ($\nu$-CN)

$^1$H-NMR-Spektrum: (d$_6$-DMSO, TMS als interner Standard)

$\delta$ = 3.17 (m) (2 x $CH_2$) 4 H,
3.50 - 4.00 (m) (2 x $CH_2$, 2 x $OCH_2$) 8 H,
4.32 (m) (CH) 1 H,
4.80 (s) ($CH_2$) 2 H,
6.57 - 7.73 (m) (Aromaten-H) 12 H,
7.87 (s) (Aromaten-H) 1 H,
8.40 (s) (Aromaten-H) 1 H ppm.

Beispiel 49

cis-1-Cyanimino-methoxy-methylen-4-[4[[2-(2,4-dichlorphenyl)-2-(1H-1,2,4-triazol-1-ylmethyl)-1,3-dioxolan-4-yl]methoxy]-phenyl]-piperazin

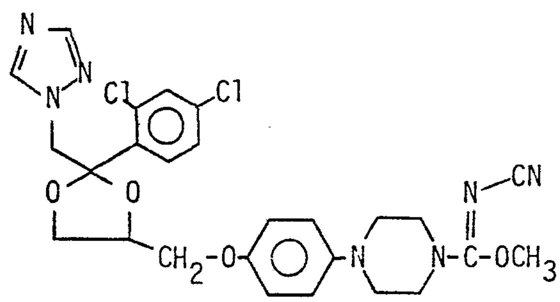

6.35 g (10 mmol) cis-1-Cyanimino-phenoxy-methylen-4-[4[[2-(2,4-dichlorphenyl)-2-(1H-1,2,4-triazol-1-ylmethyl)-1,3-dioxolan-4-yl]methoxy]-phenyl]-piperazin (Beispiel 48) werden mit 0.57 g (11 mmol) Natriummethylat in 50 ml Methanol 12 h bei Raumtemperatur umgesetzt. Nach dem Einengen des Lösungsmittels wird der Rückstand aus Essigester umkristallisiert.

Farblose Kristalle vom Schmelzpunkt 87 - 88 °C

Rf = 0.72 (Essigester/$CH_3OH$ 80 : 20)

$C_{26}H_{27}Cl_2N_7O_4$ (573) Ber.: C 54.55 H 4.75 N 17.13
Gef.: C 54.85 H 4.99 N 16.70

IR ($cm^{-1}$): 2200 ($\nu$-CN)

$^1$H-NMR-Spektrum: (d$_6$-DMSO, TMS als interner Standard)

$\delta$ = 3.13 (m) (2 x $C\underline{H}_2$) 4 H,
3.60 - 4.11 (m) (2 x $C\underline{H}_2$, 2 x $OC\underline{H}_2$) 8 H,
3.95 (s) ($OC\underline{H}_3$) 3 H,
4.37 (m) ($C\underline{H}$) 1 H,
4.83 (s) ($C\underline{H}_2$) 2 H,
6.77 - 7.80 (m) (Aromaten-$\underline{H}$) 7 H,
7.93 (s) (Aromaten-$\underline{H}$) 1 H,
8.43 (s) (Aromaten-$\underline{H}$) 1 H ppm.

Beispiel 50

cis-1-Cyanimino-methoxy-methylen-4-[4-[[2-(2,4-dichlorphenyl)-2-(1H-imidazol-1-ylmethyl)-1,3-dioxolan-4-yl] methoxy]-phenyl]-piperazin

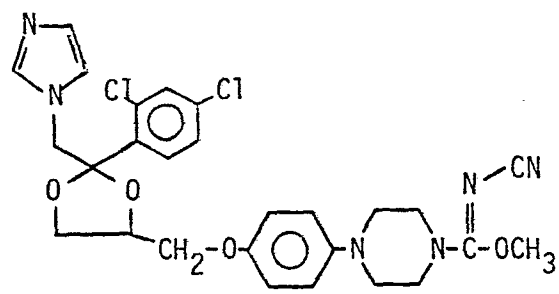

8.0 g (12.6 mmol) cis-1-Cyanimino-phenoxy-methylen-4-[4[[2-(2,4-dichlorphenyl)-2-(1H-imidazol-1-ylmethyl)-1,3-dioxolan-4-yl] methoxy]-phenyl]-piperazin (Beispiel 45) werden mit 2.1 ml (11.5 mmol) einer 5,5 molar Natriummethylatlösung in 80 ml Methanol 12 h bei Raumtemperatur umgesetzt. Nach dem Einengen der Reaktionslösung wird der Rückstand aus Isopropanol/Diisopropylether (80 : 20) umkristallisiert.

Farblose Kristalle vom Schmelzpunkt 129 - 131 °C

Rf = 0.6 (Essigester/$CH_3OH$ 80 : 20)

| | | | | |
|---|---|---|---|---|
| $C_{27}H_{28}Cl_2N_6O_4$ (572) | Ber.: | C 56.75 | H 4.94 | N 14.71 |
| | Gef.: | C 56.91 | H 5.12 | N 14.60 |

IR ($cm^{-1}$): 2200 ($\nu$-CN)

$^1H$-NMR-Spektrum: (CDCl$_3$, TMS als interner Standard)

$\delta$ = 3.1 (m) (2 x $CH_2$) 4 H,
3.60 - 4.07 (m) (2 x $OCH_2$, 2 x $CH_2$) 8 H,
4.00 (s) ($OCH_3$) 3 H,
4.37 (m) (CH) 1 H,
4.43 (s) ($CH_2$) 2 H,
6.70 - 7.73 (m) (Aromaten-H) 10 H ppm.

0152596

LUDWIG HEUMANN & CO · GMBH

8500 Nürnberg

---

Neue 1,3-Dioxolanylderivate, Verfahren zu ihrer Herstellung, ihre Verwendung und diese Verbindungen enthaltendes Arzneimittel

---

## P A T E N T A N S P R U C H E

1. 1,3-Dioxolanylderivate der allgemeinen Formel I

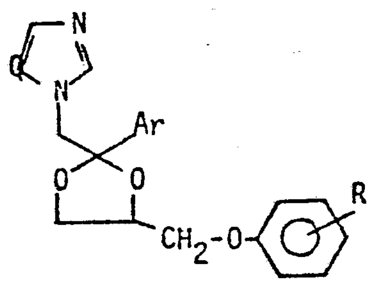

(I)

in der Q für CH oder N steht, Ar eine unsubstituierte oder mit 1 bis 3 Halogenatomen substituierte Phenylgruppe bedeutet und R

a) einen Rest der allgemeinen Formel II

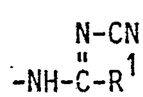

(II)

in der $R^1$ eine unsubstituierte oder substituierte Aminogruppe oder einen gegebenenfalls substituierten Pyrrolidin-, Piperidin-,

Morpholin- oder Piperazinring oder eine Alkoxy-, Phenoxy- oder Thioniedrigalkylgruppe bedeutet,

b) einen Rest der allgemeinen Formel III

$$-NH-R^2 \qquad (III)$$

in der $R^2$ für einen ein- oder zweifachsubstiuierten 5- oder 6-Ringheterocyclus aus der Gruppe 1,2,4-Triazole, 1,2,4-Oxadiazole und Triazine steht, wobei der Heterocyclus mit dem Stickstoffatom des Restes III über ein Kohlenstoffatom verbunden ist,

c) einen Rest der allgemeinen Formel IV

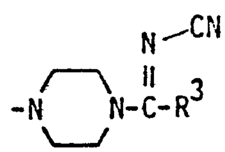

(IV)

in der $R^3$ für eine unsubstituierte oder substituierte Aminogruppe oder einen gegebenenfalls substituierten Pyrrolidin-, Piperidin-, Morpholin- oder Piperazinring oder eine Alkoxy-, Phenoxy- oder Thioniedrigalkylgruppe steht, oder

d) einen Rest der allgemeinen Formel V

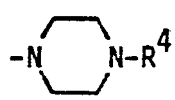

(V)

in der $R^4$ einen ein- oder zweifachsubstituierten 5- oder 6-Ringheterocyclus aus der Gruppe 1,2,4-Triazole, 1,2,4-Oxadiazole und Triazine bedeutet, wobei der Heterocyclus mit dem Stickstoffatom des Piperazinrings über ein Kohlenstoffatom verbunden ist, bedeutet,

sowie die physiologisch annehmbaren Hydrate und Salze davon.

2. 1-Cyanimino-methylthio-methylen-4-[4-[[2-(2,4-dichlorphenyl)-2-(1H-imidazol-1-ylmethyl)-1,3-dioxolan-4-yl]-methoxy]-phenyl]-piperazin und dessen physiologisch verträgliche Säureadditionssalze.

3. N'-[4-[2-(2,4-Dichlorphenyl)-2-(1H-imidazol-1-ylmethyl)-1,3-dioxolan-4-ylmethoxy]-phenyl]-N"-cyanopropylisoharnstoff und dessen physiologisch verträgliche Säureadditionssalze.

4. N'-[4-[2-(2,4-Dichlorphenyl)-2-(1H-imidazol-1-ylmethyl)-1,3-dioxolan-4-ylmethoxy]-phenyl]-N"-cyano-n-butylisoharnstoff und dessen physiologisch verträgliche Säureadditionssalze.

5. Verfahren zur Herstellung der Verbindungen nach den Ansprüchen 1 bis 4, dadurch g e k e n n z e i c h n e t , daß man

$a_1$) zur Herstellung einer Verbindung der allgemeinen Formel I, in der R für eine Gruppe der allgemeinen Formel II

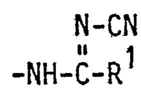

(II)

und $R^1$ für eine Thiomethyl- oder Phenoxygruppe steht, eine Verbindung der allgemeinen Formel VI

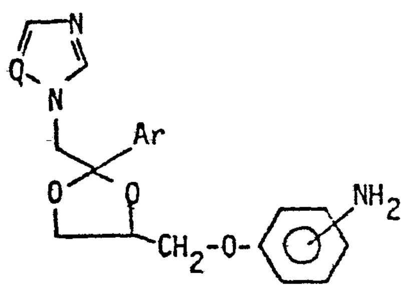

(VI)

mit einer Verbindung der allgemeinen Formel VII

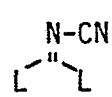

(VII)

in der L eine Thiomethyl- oder Phenoxygruppe als Austrittsgruppe bedeutet, in einem Lösungsmittel zu einer erfindungsgemäßen Verbindung der allgemeinen Formel Ia

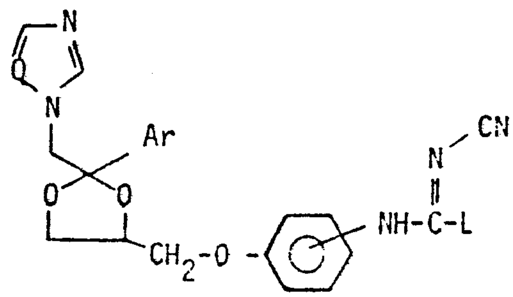

(Ia)

in der L eine Thiomethyl- oder Phenoxygruppe bedeutet, umsetzt;

oder daß man

$a_2$) zur Herstellung einer Verbindung der allgemeinen Formel I, in der R für eine Gruppe der allgemeinen Formel II

$$-NH-\overset{\overset{N-CN}{\|}}{C}-R^1 \qquad (II)$$

und $R^1$ für eine unsubstituierte oder substituierte Aminogruppe steht, eine Verbindung der allgemeinen Formel Ia mit einer entsprechend substituierten Aminoverbindung in einem Lösungsmittel umsetzt;

oder daß man

$a_3$) zur Herstellung einer Verbindung der allgemeinen Formel I, in der R für eine Gruppe der allgemeinen Formel II

$$-NH-\overset{\overset{N-CN}{\|}}{C}-R^1 \qquad (II)$$

und $R^1$ für eine Alkoxygruppe steht, eine Verbindung der allgemeinen Formel Ia mit einem Alkalialkoholat in dem entsprechenden Alkohol als Lösungsmittel umsetzt;

oder daß man

$a_4$) zur Herstellung einer Verbindung der allgemeinen Formel I, in der R für eine Gruppe der allgemeinen Formel II steht,

$$-NH-\overset{N-CN}{\overset{\|}{C}}-R^1 \qquad (II)$$

und $R^1$ die in Anspruch 1 angegebene Bedeutung besitzt, eine Verbindung der allgemeinen Formel VIII

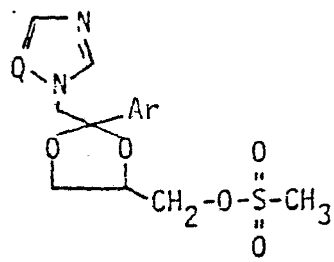

(VIII)

mit einer Verbindung der allgemeinen Formel IX

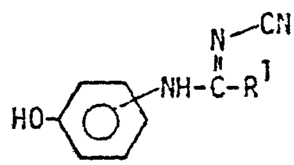

(IX)

in der $R^1$ die in Anspruch 1 angegebene Bedeutung besitzt, umsetzt;

oder daß man

$b_1$) zur Herstellung einer Verbindung der allgemeinen Formel I, in der R für eine Gruppe der allgemeinen Formel III steht

$$-NH-R^2 \qquad (III)$$

und $R^2$ die in Anspruch 1 angegebene Bedeutung besitzt, eine Verbindung der allgemeinen Formel Ia mit einem Hydrazinderivat, Hydroxylamin oder Guanidin in einem Lösungsmittel umsetzt;

oder daß man

$b_2$) zur Herstellung einer Verbindung der allgemeinen Formel I, in der R für eine Gruppe der allgemeinen Formel III

$$-NH-R^2 \qquad \text{(III)}$$

steht und $R^2$ die in Anspruch 1 angegebene Bedeutung besitzt, eine Verbindung der allgemeinen Formel VIII

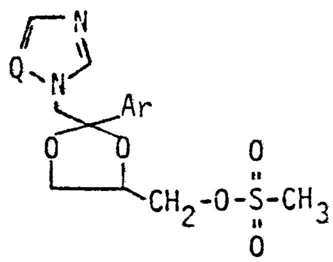

(VIII)

mit einer Verbindung der allgemeinen Formel X

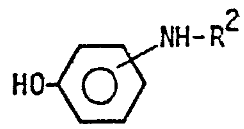

(X)

in der $R^2$ die in Anspruch 1 angegebene Bedeutung besitzt, umsetzt;

oder daß man

$c_1$) zur Herstellung einer Verbindung der allgemeinen Formel I, in der R für eine Gruppe der allgemeinen Formel IV

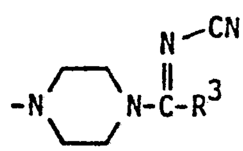

(IV)

steht und $R^3$ für eine Thiomethyl- oder Phenoxygruppe steht, eine Verbindung der allgemeinen Formel XI

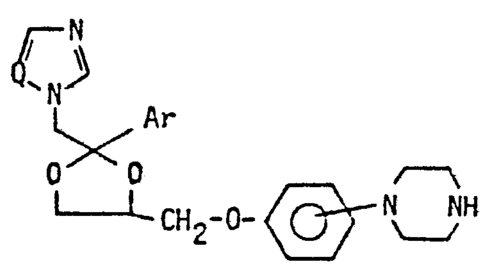

(XI)

mit einer Verbindung der allgemeinen Formel VII

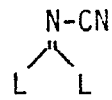

(VII)

in der L eine Thiomethyl- oder Phenoxygruppe als Austrittsgruppe bedeutet, in einem Lösungsmittel zu einer erfindungsgemäßen Verbindung der allgemeinen Formel Ib

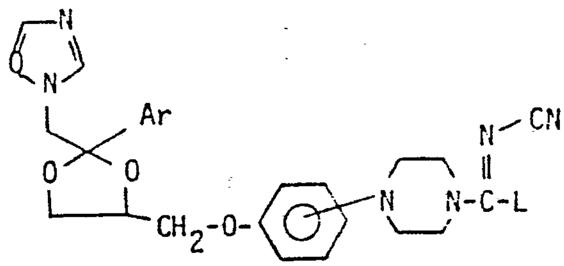

(Ib)

in der L eine Thiomethyl- oder Phenoxygruppe bedeutet, umsetzt;

oder daß man

$c_2$) zur Herstellung einer Verbindung der allgemeinen Formel I, in der R für eine Gruppe der allgemeinen Formel IV

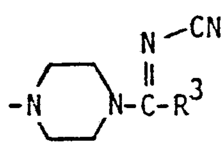

(IV)

steht und $R^3$ für eine unsubstituierte oder substituierte Aminogruppe steht, eine Verbindung der allgemeinen Formel Ib mit einer entsprechend substituierten Aminoverbindung in einem Lösungsmittel umsetzt;

oder daß man

$c_3$) zur Herstellung einer Verbindung der allgemeinen Formel I, in der R für eine Gruppe der allgemeinen Formel IV und $R^3$ für eine Alkoxygruppe steht, eine Verbindung der allgemeinen Formel Ib mit einem Alkalialkoholat in dem entsprechenden Alkohol als Lösungsmittel umsetzt;

oder daß man

$c_4$) zur Herstellung einer Verbindung der allgemeinen Formel I, in der R für eine Gruppe der allgemeinen Formel IV steht und $R^3$ die in Anspruch 1 angegebene Bedeutung besitzt, eine Verbindung der allgemeinen Formel VIII

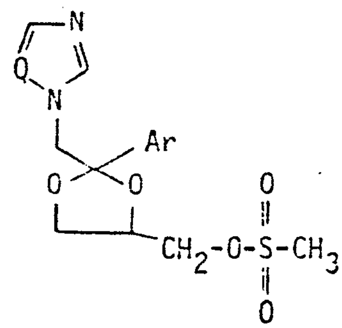

(VIII)

mit einer Verbindung der allgemeinen Formel XII

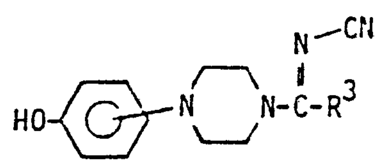

(XII)

in der $R^3$ die in Anspruch 1 angegebene Bedeutung besitzt, umsetzt;

oder daß man

$d_1$) zur Herstellung einer Verbindung der allgemeinen Formel I, in der R für eine Gruppe der allgemeinen Formel V

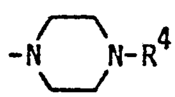

(V)

steht und $R^4$ die in Anspruch 1 angegebene Bedeutung besitzt, eine Verbindung der allgemeinen Formel Ib mit einem Hydrazinderivat, Hydroxylamin oder Guanidin in einem Lösungsmittel umsetzt;

oder daß man

$d_2$) zur Herstellung einer Verbindung der allgemeinen Formel I, in der R für eine Gruppe der allgemeinen Formel V steht und $R^4$ die

in Anspruch 1 angegebene Bedeutung besitzt, eine Verbindung der allgemeinen Formel VIII mit einer Verbindung der allgemeinen Formel XIII

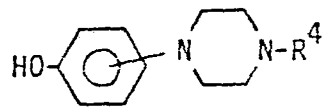

(XIII)

in der $R^4$ die in Anspruch 1 angegebene Bedeutung besitzt, umsetzt;

und daß man gegebenenfalls die in den Herstellungsstufen a) bis d) erhaltenen Verbindungen in an sich bekannter Weise in ihr physiologisch annehmbares Salz umwandelt.

6. Verwendung einer Verbindung nach den Ansprüchen 1 bis 4 zur Bekämpfung von Mykosen, Protozoen und grampositiver Bakterien.

7. Arzneimittel, dadurch gekennzeichnet, daß es eine Verbindung nach den Ansprüchen 1 bis 4 und mindestens einen inerten, pharmazeutisch annehmbaren Träger oder ein inertes, pharmazeutisch annehmbares Verdünnungsmittel enthält.

0152596

## PATENTANSPRÜCHE für AT

1. Verfahren zur Herstellung von 1,3-Dioxolanylderivaten der allgemeinen Formel I

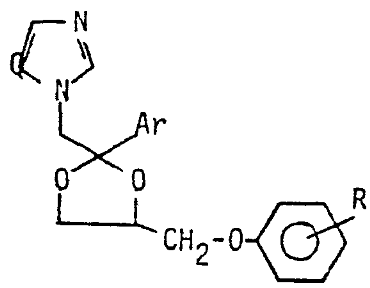

(I)

in der Q für CH oder N steht, Ar eine unsubstituierte oder mit 1 bis 3 Halogenatomen substituierte Phenylgruppe bedeutet und R

a) einen Rest der allgemeinen Formel II

$$-NH-\overset{\overset{N-CN}{\|}}{C}-R^1 \quad (II)$$

in der $R^1$ eine unsubstituierte oder substituierte Aminogruppe oder einen gegebenenfalls substituierten Pyrrolidin-, Piperidin-, Morpholin- oder Piperazinring oder eine Alkoxy-, Phenoxy- oder Thioniedrigalkylgruppe bedeutet,

b) einen Rest der allgemeinen Formel III

$$-NH-R^2 \quad (III)$$

in der $R^2$ für einen ein- oder zweifachsubstiuierten 5- oder 6-Ringheterocyclus aus der Gruppe 1,2,4-Triazole, 1,2,4-Oxadiazole und Triazine steht, wobei der Heterocyclus mit dem Stickstoffatom des Restes III über ein Kohlenstoffatom verbunden ist,

c) einen Rest der allgemeinen Formel IV

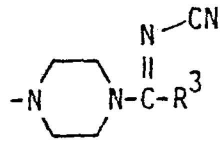

(IV)

in der $R^3$ für eine unsubstituierte oder substituierte Aminogruppe oder einen gegebenenfalls substituierten Pyrrolidin-, Piperidin-, Morpholin- oder Piperazinring oder eine Alkoxy-, Phenoxy- oder Thioniedrigalkylgruppe steht, oder

d) einen Rest der allgemeinen Formel V

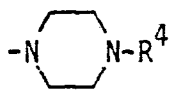

(V)

in der $R^4$ einen ein- oder zweifachsubstituierten 5- oder 6-Ringheterocyclus aus der Gruppe 1,2,4-Triazole, 1,2,4-Oxadiazole und Triazine bedeutet, wobei der Heterocyclus mit dem Stickstoffatom des Piperazinrings über ein Kohlenstoffatom verbunden ist, bedeutet,

sowie der physiologisch annehmbaren Hydrate und Salze davon, dadurch g e k e n n z e i c h n e t , daß man

$a_1$) zur Herstellung einer Verbindung der allgemeinen Formel I, in der R für eine Gruppe der allgemeinen Formel II

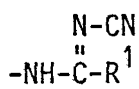

(II)

und $R^1$ für eine Thiomethyl- oder Phenoxygruppe steht, eine Verbindung der allgemeinen Formel VI

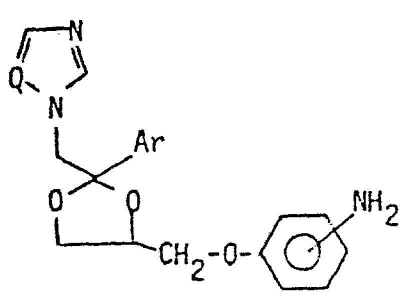

(VI)

mit einer Verbindung der allgemeinen Formel VII

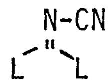 (VII)

in der L eine Thiomethyl- oder Phenoxygruppe als Austrittsgruppe bedeutet, in einem Lösungsmittel zu einer erfindungsgemäßen Verbindung der allgemeinen Formel Ia

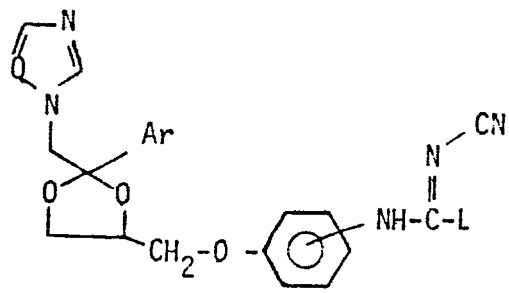 (Ia)

in der L eine Thiomethyl- oder Phenoxygruppe bedeutet, umsetzt;

oder daß man

$a_2$) zur Herstellung einer Verbindung der allgemeinen Formel I, in der R für eine Gruppe der allgemeinen Formel II

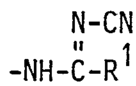 (II)

und $R^1$ für eine unsubstituierte oder substituierte Aminogruppe steht, eine Verbindung der allgemeinen Formel Ia mit einer entsprechend substituierten Aminoverbindung in einem Lösungsmittel umsetzt;

oder daß man

$a_3$) zur Herstellung einer Verbindung der allgemeinen Formel I, in der R für eine Gruppe der allgemeinen Formel II

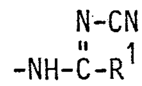

(II)

und $R^1$ für eine Alkoxygruppe steht, eine Verbindung der allgemeinen Formel Ia mit einem Alkalialkoholat in dem entsprechenden Alkohol als Lösungsmittel umsetzt;

oder daß man

$a_4$) zur Herstellung einer Verbindung der allgemeinen Formel I, in der R für eine Gruppe der allgemeinen Formel II steht,

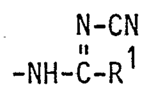

(II)

und $R^1$ die oben angegebene Bedeutung besitzt, eine Verbindung der allgemeinen Formel VIII

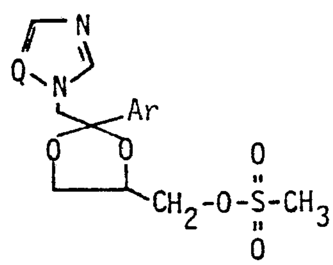

(VIII)

mit einer Verbindung der allgemeinen Formel IX

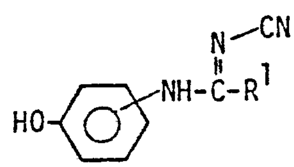

(IX)

in der $R^1$ die oben angegebene Bedeutung besitzt, umsetzt;

oder daß man

$b_1$) zur Herstellung einer Verbindung der allgemeinen Formel I, in der R für eine Gruppe der allgemeinen Formel III steht

$$-NH-R^2 \qquad (III)$$

und $R^2$ die oben angegebene Bedeutung besitzt, eine Verbindung der allgemeinen Formel Ia mit einem Hydrazinderivat, Hydroxylamin oder Guanidin in einem Lösungsmittel umsetzt;

oder daß man

$b_2$) zur Herstellung einer Verbindung der allgemeinen Formel I, in der R für eine Gruppe der allgemeinen Formel III

$$-NH-R^2 \qquad (III)$$

steht und $R^2$ die oben angegebene Bedeutung besitzt, eine Verbindung der allgemeinen Formel VIII

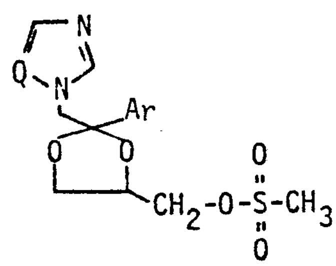

(VIII)

mit einer Verbindung der allgemeinen Formel X

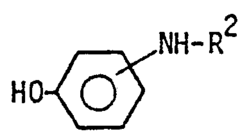

(X)

in der $R^2$ die oben angegebene Bedeutung besitzt, umsetzt;

oder daß man

$c_1$) zur Herstellung einer Verbindung der allgemeinen Formel I, in der R für eine Gruppe der allgemeinen Formel IV

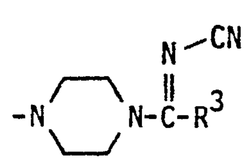

(IV)

steht und $R^3$ für eine Thiomethyl- oder Phenoxygruppe steht, eine Verbindung der allgemeinen Formel XI

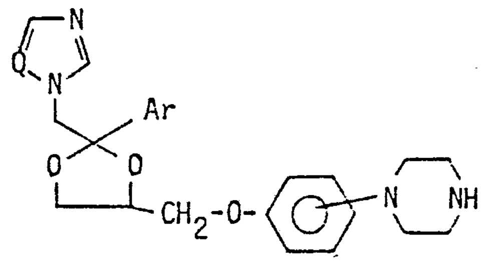

(XI)

mit einer Verbindung der allgemeinen Formel VII

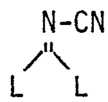

(VII)

in der L eine Thiomethyl- oder Phenoxygruppe als Austrittsgruppe bedeutet, in einem Lösungsmittel zu einer erfindungsgemäßen Verbindung der allgemeinen Formel Ib

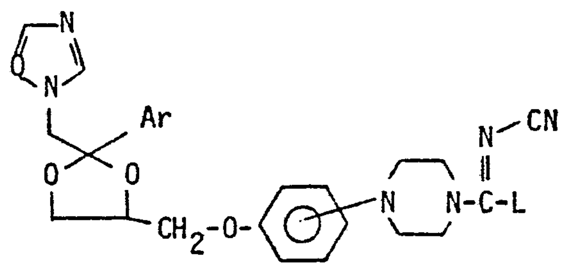

(Ib)

in der L eine Thiomethyl- oder Phenoxygruppe bedeutet, umsetzt;

oder daß man

$c_2$) zur Herstellung einer Verbindung der allgemeinen Formel I, in der R für eine Gruppe der allgemeinen Formel IV

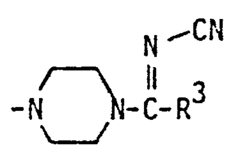

(IV)

steht und $R^3$ für eine unsubstituierte oder substituierte Aminogruppe steht, eine Verbindung der allgemeinen Formel Ib mit einer entsprechend substituierten Aminoverbindung in einem Lösungsmittel umsetzt;

oder daß man

$c_3$) zur Herstellung einer Verbindung der allgemeinen Formel I, in der R für eine Gruppe der allgemeinen Formel IV und $R^3$ für eine Alkoxygruppe steht, eine Verbindung der allgemeinen Formel Ib mit einem Alkalialkoholat in dem entsprechenden Alkohol als Lösungsmittel umsetzt;

oder daß man

$c_4$) zur Herstellung einer Verbindung der allgemeinen Formel I, in der R für eine Gruppe der allgemeinen Formel IV steht und $R^3$ die oben angegebene Bedeutung besitzt, eine Verbindung der allgemeinen Formel VIII

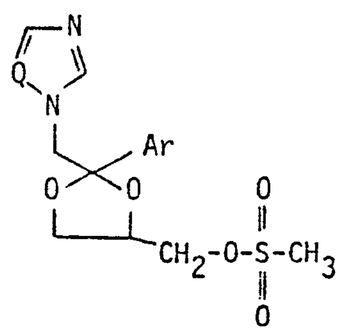

(VIII)

mit einer Verbindung der allgemeinen Formel XII

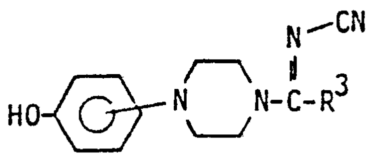

(XII)

in der $R^3$ die oben angegebene Bedeutung besitzt, umsetzt;

oder daß man

$d_1$) zur Herstellung einer Verbindung der allgemeinen Formel I, in der R für eine Gruppe der allgemeinen Formel V

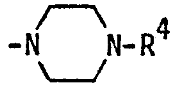

(V)

steht und $R^4$ die oben angegebene Bedeutung besitzt, eine Verbindung der allgemeinen Formel Ib mit einem Hydrazinderivat, Hydroxylamin oder Guanidin in einem Lösungsmittel umsetzt;

oder daß man

$d_2$) zur Herstellung einer Verbindung der allgemeinen Formel I, in der R für eine Gruppe der allgemeinen Formel V steht und

$R^4$ die oben angegebene Bedeutung besitzt, eine Verbindung der allgemeinen Formel VIII mit einer Verbindung der allgemeinen Formel XIII

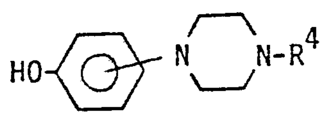

(XIII)

in der $R^4$ die oben angegebene Bedeutung besitzt, umsetzt;

und daß man gegebenenfalls die in den Herstellungsstufen a) bis d) erhaltenen Verbindungen in an sich bekannter Weise in ihr physiologisch annehmbares Salz umwandelt.

2. Verfahren nach Anspruch 1 zur Herstellung von 1-Cyanimino-methylthio-methylen-4-[4-[[2-(2,4-dichlorphenyl)-2-(1H-imidazol-1-ylmethyl)-1,3-dioxolan-4-yl]-methoxy]-phenyl]-piperazin und dessen physiologisch verträgliche Säureadditionssalze.

3. Verfahren nach Anspruch 1 zur Herstellung von N'-[4-[2-(2,4-Dichlorphenyl)-2-(1H-imidazol-1-ylmethyl)-1,3-dioxolan-4-ylmethoxy]-phenyl]-N"-cyanopropylisoharnstoff und dessen physiologisch verträgliche Säureadditionssalze.

4. Verfahren nach Anspruch 1 zur Herstellung von N'-[4-[2-(2,4-Dichlorphenyl)-2-(1H-imidazol-1-ylmethyl)-1,3-dioxolan-4-ylmethoxy]-phenyl]-N"-cyano-n-butylisoharnstoff und dessen physiologisch verträgliche Säureadditionssalze.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0152596

Nummer der Anmeldung

EP 84115443.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2/3 - 0 050 298 (HOECHST)<br>* Formel I *<br>-- | 1 | C 07 D 405/06<br>C 07 D 405/14<br>C 07 D 413/14<br>A 61 K 31/41 |
| A | GB - A - 2 098 607 (CIBA)<br>* Formel I *<br>-- | 1 | |
| A | DE - A1 - 2 940 133 (JANSSEN)<br>* Formel I *<br>-- | 1 | |
| A | GB - A - 1 594 859 (JANSSEN)<br>* Formel I *<br>-- | 1 | |
| A | EP - B1 - 0 006 712 (JANSSEN)<br>* Anspruch 1 *<br>-- | 1 | **RECHERCHIERTE SACHGEBIETE (Int. Cl.4)** |
| P,A | AT - E - 5 140 (JANSSEN)<br>* Formel I *<br>-- | 1 | C 07 D 405/00<br>C 07 D 413/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5,7

Unvollständig recherchierte Patentansprüche: –

Nicht recherchierte Patentansprüche: 6

Grund für die Beschränkung der Recherche:

(Art. 52 (4) EPÜ; Verfahren zur therapeutischen Behandlung des menschlichen oder tierischen Körpers)

| Recherchenort | Abschlußdatum der Recherche | Prüfer |
|---|---|---|
| WIEN | 15-05-1985 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

Europäisches Patentamt

# EUROPÄISCHER TEILRECHERCHENBERICHT

0152596
Nummer der Anmeldung

EP 84115443.8

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl. 4) |
|---|---|---|---|
| A | AT - E - 1 709 (JANSSEN)<br>* Formel I *<br>---- | 1 | |

RECHERCHIERTE SACHGEBIETE (Int. Cl. 4)

EPA Form 1505.3 06.78